# EUROPEAN PATENT SPECIFICATION

(11) **EP 3 803 400 B1**
(45) Date of publication and mention of the grant of the patent: **09.08.2023**
(21) Application number: 19728095.1
(22) Date of filing: 06.06.2019
(51) Int. Cl.: G01N 33/569, G01N 33/68, C07K 14/35

(54) **IN VITRO METHOD FOR THE DIAGNOSIS OR DETECTION OF NON-TUBERCULOUS MYCOBACTERIA**
IN-VITRO-VERFAHREN ZUR DIAGNOSE ODER DETEKTION VON NICHT TUBERKULÖSEN MYKOBAKTERIEN
PROCÉDÉ IN VITRO POUR LE DIAGNOSTIC OU LA DÉTECTION D'INFECTIONS MYCOBACTÉRIENNES NON TUBERCULEUSES

(30) Priority: 06.06.2018 EP 18382403
(43) Date of publication of application: 14.04.2021
(73) Proprietor: Fundació Institut d'Investigació en Ciències de la Salut Germans Trias i Pujol, 08916 Badalona (ES); Ohio State Innovation Foundation, Columbus, OH 43201 (US); Consorcio Centro de Investigación Biomédica en Red M.P., 28029 Madrid (ES)
(72) Inventor: DOMÍNGUEZ BENÍTEZ, José Antonio, 08916 Badalona (ES); LATORRE RUEDA, Irene, 08916 Badalona (ES); VILLAR-HERNÁNDEZ, Raquel, 08916 Badalona (ES); TORRELLES, Jordi, San Antonio, TX 78256 (US)
(74) Representative: Hoffmann Eitle
(86) International application number: PCT/EP2019/064885
(87) International publication number: WO 2019/234196

(56) References cited:
- WO-A1-02/066985
- CN-A- 105 866 406
- S. KITADA ET AL: "Use of Glycopeptidolipid Core Antigen for Serodiagnosis of Mycobacterium avium Complex Pulmonary Disease in Immunocompetent Patients", CLINICAL AND VACCINE IMMUNOLOGY, vol. 12, no. 1, January 2005 (2005-01), pages 44-51, XP055498930, US ISSN: 1556-6811, DOI: 10.1128/CDLI.12.1.44-51.2005
- J. S SCHOREY ET AL: "The mycobacterial glycopeptidolipids: structure, function, and their role in pathogenesis", GLYCOBIOLOGY, vol. 18, no. 11, 22 August 2008 (2008-08-22), pages 832-841, XP055498365, US ISSN: 0959-6658, DOI: 10.1093/glycob/cwn076
- BIET ET AL: "Lipopentapeptide induces a strong host humoral response and distinguishes Mycobacterium avium subsp. paratuberculosis from M. avium subsp. avium", VACCINE, ELSEVIER, AMSTERDAM, NL, vol. 26, no. 2, 20 November 2007 (2007-11-20), pages 257-268, XP022394785, ISSN: 0264-410X, DOI: 10.1016/J.VACCINE.2007.10.059
- SCHWARZ C: "Glycopeptidolipids of the Mycobacterium abscessus cell wall are immunodominant antigens and represent potential targets for a diagnostic assay", JOURNAL OF CYSTIC FIBROSIS 20180601 ELSEVIER B.V. NLD, vol. 17, no. Supplement 3, 1 June 2018 (2018-06-01), XP009507399, ISSN: 1873-5010
- STOUT JASON E ET AL: "Update on pulmonary disease due to non-tuberculous mycobacteria", INTERNATIONAL JOURNAL OF INFECTIOUS DISEASES, INTERNATIONAL SOCIETY FOR INFECTIOUS DISEASES, HAMILTON, CA, vol. 45, 11 March 2016 (2016-03-11), pages 123-134, XP029508389, ISSN: 1201-9712, DOI: 10.1016/J.IJID.2016.03.006
- LOPEZ-VARELA E ET AL: "Non-tuberculous mycobacteria in children: Muddying the waters of tuberculosis diagnosis", THE LANCET. RESPIRATORY MEDICINE, ELSEVIER, OXFORD, vol. 3, no. 3, March 2015 (2015-03), pages 244-256, XP009507337, ISSN: 2213-2600, DOI: 10.1016/S2213-2600(15)00062-4 [retrieved on 2015-03-09]
- I. LATORRE ET AL: "Evaluating the non-tuberculous mycobacteria effect in the tuberculosis infection diagnosis", EUROPEAN RESPIRATORY JOURNAL., vol. 35, no. 2, 1 February 2010 (2010-02-01), pages 338-342, XP055498654, DK ISSN: 0903-1936, DOI: 10.1183/09031936.00196608
- VANKAYALAPATI R ET AL: "Cytokine profiles in immunocompetent persons infected with Mycobacterium avium complex.", THE JOURNAL OF INFECTIOUS DISEASES 01 FEB 2001, vol. 183, no. 3, 1 February 2001 (2001-02-01), pages 478-484, XP055498753, ISSN: 0022-1899
- PAI M ET AL: "Interferon-gamma assays in the immunodiagnosis of tuberculosis: a systematic review", LANCET INFECTIOUS DISE, ELSEVIER LTD, US, vol. 4, no. 12, December 2004 (2004-12), pages 761-776, XP004812171, ISSN: 1473-3099, DOI: 10.1016/S1473-3099(04)01206-X
- MAO-SHUI WANG ET AL: "The performance of interferon-gamma release assay in nontuberculous mycobacterial diseases: a retrospective study in China", BMC PULMONARY MEDICINE, BIOMED CENTRAL LTD, LONDON, UK, vol. 16, no. 1, 25 November 2016 (2016-11-25), pages 1-5, XP021239181, DOI: 10.1186/S12890-016-0320-3

## Description

### FIELD OF THE INVENTION

The present invention is defined by the appended claims and relates to the medical and/or veterinary field. More specifically, the present invention refers to an *in vitro* method (hereinafter method of the invention) for detecting immune response against species of non-tuberculous mycobacteria (NTM). Consequently, it can be used in the diagnosis of NTM infections. Particularly, the method of the invention can be used for the diagnosis of diseases directly caused by NTM infections for example: pulmonary infections, lymphadenitis and skin and soft tissue infections. Specifically, it can be used to identify NTM infections in children diagnosed with cystic fibrosis, in patients suffering from chronic respiratory diseases, including chronic obstructive pulmonary disease (COPD) and, among others, in immunosuppressed adults. On the other hand, the method of the invention can be used for the differential diagnosis of NTM infections vs. tuberculosis (TB) infections in children with lymphadenopathy. Moreover, the method of the invention can be also used for differentiating NTM immune response from latent *Mycobacterium* tuberculosis infection (LTBI), allowing a more accurate diagnosis of LTBI, particularly in the pediatric population.

### BACKGROUND OF THE INVENTION

Historically, reported human infections due to *Mycobacterium spp.* were due almost exclusively to *Mycobacterium tuberculosis.* The extensive public impact of this infection is legendary. More recently, other species of *Mycobacterium spp.* causing TB-like clinical disease have been identified and are increasingly recognized worldwide. These organisms are referred as NTM. NTM can cause a broad range of infections. Although over 150 different species of NTM have been described, pulmonary infections are most commonly due to *Mycobacterium avium* complex (MAC), *Mycobacterium kansasii,* and *Mycobacterium abscesses.* NTM infections are also of particular importance in patients with chronic respiratory diseases (cystic fibrosis, COPD, etc.). In addition to pulmonary infections NTM can cause infections in other localizations. In children, NTM cause four main clinical syndromes: lymphadenitis, skin and soft tissue infection, pulmonary diseases (predominantly in children with underlying pulmonary conditions), and disseminated disease (in immune-compromised children).

The identification of NTM in pulmonary specimens does not always equate with active infection. A prolonged course of therapy with a combination of drugs is then required. In addition, recurrent infection with new strains of mycobacteria or a relapse of infection is not uncommon, being appropriate in some selected cases a surgical resection. Nowadays, not supportive definite diagnostic methods are still available.

Thus, having a diagnostic method for the identification of patients who really have a NTM infection would help to prescribe the adequate treatment to those that really need it, consequently reducing unnecessary therapies, with the subsequent reduction in antibiotic resistance, and costs of patient treatment.

Particularly, there is a real need of reliable methods for the diagnosis of diseases directly caused by NTM infections. For example, pulmonary infections, lymphadenitis and skin and soft tissue infections are the most commonly described diseases, which are caused by NTM infections. Between them, it could be highlighted: i) The colonization/infection of the airway respiratory tract by NTM in children diagnosed with cystic fibrosis and ii) NTM infection of patients suffering chronic respiratory diseases and immunosupressed patients.

it is important to consider that diagnostic methods helping in the distinction between colonization and infection would be very relevant, particularly in the case of patients suffering chronic respiratory diseases and immunosupressed patients. Accuracy in the diagnosis is needed because NTM infection requires a prolonged course of therapy with a combination of drugs, being appropriate in some selected cases a surgical lung resection. Nowadays, no supportive and definite methods for the diagnosis of NTM infections are still available. The conventional methods used today for the diagnosis of NTM infections, basically the analysis of the sputum culture combined with clinical criteria, cannot help in distinguishing between colonization and active infection. Thus, having a diagnostic method aimed at identifying patients who really have a NTM infection (not just colonization) would help to prescribe the adequate treatment for said patients. This would also reduce unnecessary therapies and, consequently, adverse effects, reducing also antibiotic resistance, and costs of patient treatment. The administration of the adequate treatment would improve also the quality of life of patients, reducing also the number of occasions that they attend to hospitals.

Moreover, there is a real need of reliable methods focused on the differential diagnosis between NTM and LTBI in children with lymphadenopathy, when there is a suspicion of active TB. The treatment of the lymphadenopathy in children requires the extraction of the lymphadenopathy by surgery, and also the prescription of specific treatment that is different if the lymphadenopathy is due to NTM infections (mainly *M*. *avium*) or *M*. *tuberculosis.*

Additionally, there is a real need of diagnosis methods aimed at diagnosing NTM infections in situations where the NTM immune response confuses tuberculin skin tests (TST) results. Diagnosing LTBI in children is very important because the risk of progression to severe active TB is high, especially in young children. The guidelines recommend using the TST and the interferon gamma release assay (IGRAs). IGRAs are more specific tests than TST, given that the antigens used for stimulating the T-cells are only present in *M*. *tuberculosis.* However, due the high risk of progression in case of real infection and the severity of the TB in children, when a positive TST and a negative IGRA is reported, the patient is frequently considered as infected and is treated as a LTBI patient, especially in non BCG-vaccinated children. The inventors of the present invention have observed that a large number of patients are not properly diagnosed as having LTBI, because some of these patients are responding to the TST due to cross-reaction with previous sensitization with NTM. Consequently, there is a real need of identifying adequately the NTM infections in order to administrate the adequate therapy, avoiding unnecessary treatments due to false positive TST results. In fact, the use of an *in-vitro* test able to identify the sensitization with NTM would allow the identification of TST false-positive results, and consequently reduce the number of unnecessary LTBI treatments in children. Thus, the social alarm and the cost of the overall management of the cases (patient treatment, contact-tracing studies, LTBI screenings in schools) would be considerably reduced.

Consequently, as explained above, diagnosing differentially NTM is a clear unmet medical need. Nevertheless, nowadays there is not a test in the market using specific antigens for T-cell stimulation for diagnosing specifically NTM infections. While the TST and the IGRAs are approved diagnostic tests for identifying LTBI, there is no procedure for NTM infections. WO02/066985 discloses that the invariant lipid core of a Mycobacterium avium GPL derived from the cell membrane is used as an active ingredient in an immunological assay to distinguish between tuberculosis and NTM infections, in particular infections by Mycobacterium avium complex (MAC), which will influence the choice of treatment.

Kitada et al. 2005 (Clin Diagn Lab Immunol 12(1):44-51) discloses that the GPL core antigen from nontuberculous mycobacteria (NTM) Mycobacterium avium complex (MAC) is used as antigen in an enzyme immunoassay to diagnose pulmonary MAC disease.

### DESCRIPTION OF THE INVENTION

### Brief description of the invention

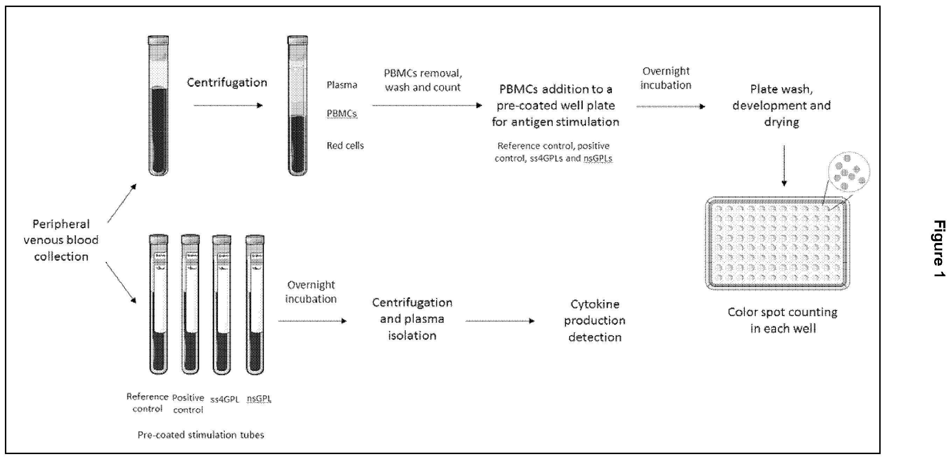

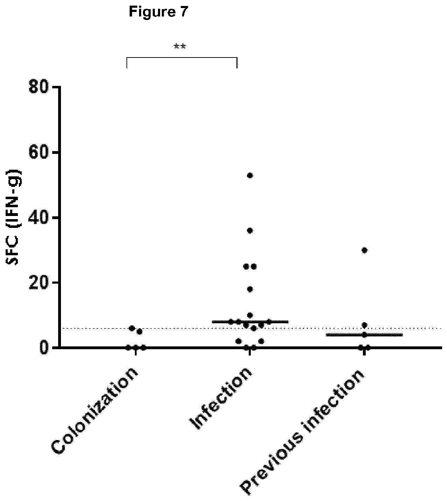

The inventors of the present invention, by using precisely *M*. *avium* nsGPLs antigens, have been able to identify non only infections caused by *M. avium* but also infections caused by any NTM; in particular by any of the following bacterial species selected from the list consisting of: *Mycobacterium abcessus, Mycobacterium chimaera, Mycobacterium fortuitum, Mycobacterium intracellulare, Mycobacterium malmoense, Mycobacterium xenopi, Mycobacterium chelonae, Mycobacterium farcinogenes, Mycobacterium Habana, Mycobacterium hominis, Mycobacterium paratuberculosis, Mycobacterium peregrinun, Mycobacterium porcinum, Mycobacterium senegalense, Mycobacterium smegmatis, Mycobacterium scrofulaceum* and *Mycobacterium simiae;* more particularly by any of the following bacterial species selected from the list consisting of: *Mycobacterium abcessus, Mycobacterium avium, Mycobacterium chimaera, Mycobacterium fortuitum, Mycobacterium intracellulare, Mycobacterium malmoense* and *Mycobacterium xenopi.* It is noted that tables 1 and 5 to 7, clearly show positive results obtained after stimulation with non-serovar specific Glycopeptidelipids (nsGPLs) such as ns4GPL or ns8GPL, or *Mycobacterium smegmatis.* It is noted that both chemical structures, ns4GPL or ns8GPL, are chemically identical. It is further noted that, as shown in figures 1 to 7, stimulation with such non-serovar specific Glycopeptidelipids have provided positive results for *Mycobacterium abcessus, Mycobacterium avium, Mycobacterium chimaera, Mycobacterium intracellulare, Mycobacterium malmoense* and *Mycobacterium xenopi.* As regards *Mycobacterium fortuitum,* negative results are shown in the present examples, since the patients from which the samples were taken, were colonized with this bacteria without producing infection (these results are in line with the results shown in figure 7 where it is clear that the non-serovar specific Glycopeptidelipids of the invention, are useful for differentiating between bacterial colonization of an individual and infection (clinical manifestation of the disease)).

Thus, the present invention gives rise to a versatile tool for the identification of infections caused by any NTM, not only by *M. avium.* This technical effect has been unexpectedly achieved in the present invention, as shown in tables 1 and 5 to 7, by using, precisely, nsGPLs of Formula I derived from *M. avium.* Thus, the present invention , as defined by the appended claims, is focused on detecting infections caused not only by *M*. *avium* but caused by any NTM, using the antigen of Formula I, or an antigen comprising the chemical structure of formula I, or any isomer thereof:

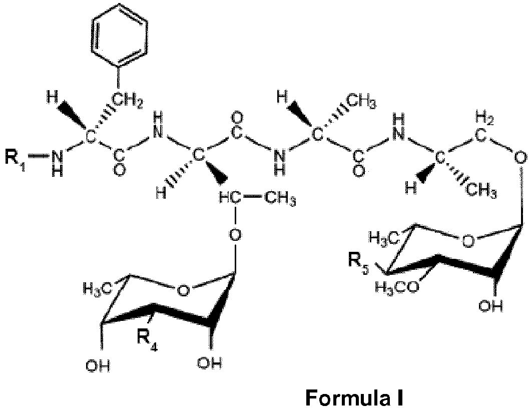

Wherein **R₁** is

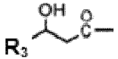

Wherein **R₃** is an alkyl or alkenyl chain having from 1 to 34 carbon atoms.
Wherein **R₄** is -OH or -OCH₃
Wherein **R₅** is -OH or -OCH₃

It is, however, noted that identification of infections caused by any NTM, in particular by any of the following bacterial species selected from the list consisting of: *Mycobacterium abcessus, Mycobacterium chimaera, Mycobacterium fortuitum, Mycobacterium intracellulare, Mycobacterium malmoense, Mycobacterium xenopi, Mycobacterium chelonae, Mycobacterium farcinogenes, Mycobacterium Habana, Mycobacterium hominis, Mycobacterium paratuberculosis, Mycobacterium peregrinun, Mycobacterium porcinum, Mycobacterium senegalense, Mycobacterium smegmatis, Mycobacterium scrofulaceum* and *Mycobacterium simiae;* is not only achieved by antigens consisting or comprising formula I, but by any nsGPLs of any of the following bacteria: *Mycobacterium abcessus, Mycobacterium chimaera, Mycobacterium fortuitum, Mycobacterium intracellulare, Mycobacterium malmoense, Mycobacterium xenopi* or *Mycobacterium smegmatis.*

Therefore, an alternative aspect disclosed herein but not part of the invention is focused on detecting infections caused by any NTM, using as an antigen any nsGPLs, or an antigen comprising such nsGPLs, of any of the following bacteria selected from the group consisting of: *Mycobacterium abcessus, Mycobacterium chimaera, Mycobacterium fortuitum, Mycobacterium intracellulare, Mycobacterium malmoense, Mycobacterium xenopi* or *Mycobacterium smegmatis.* In particular, such nsGPLs are capable of detecting infections caused by any of the following bacterial species selected from the list consisting of: *Mycobacterium abcessus, Mycobacterium chimaera, Mycobacterium fortuitum, Mycobacterium intracellulare, Mycobacterium malmoense, Mycobacterium xenopi, Mycobacterium chelonae, Mycobacterium farcinogenes, Mycobacterium Habana, Mycobacterium hominis, Mycobacterium paratuberculosis, Mycobacterium peregrinun, Mycobacterium porcinum, Mycobacterium senegalense, Mycobacterium smegmatis, Mycobacterium scrofulaceum* and *Mycobacterium simiae;* more particularly by any of the following bacterial species selected from the list consisting of: *Mycobacterium abcessus, Mycobacterium avium, Mycobacterium chimaera, Mycobacterium fortuitum, Mycobacterium intracellulare, Mycobacterium malmoense* and *Mycobacterium xenopi.* Preferably, such nsGPLs is an antigen of Formula II, or an antigen comprising the chemical structure of formula II, or any isomer thereof:

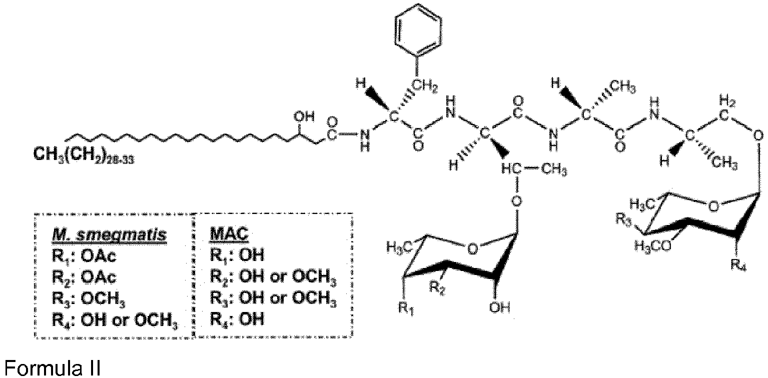

In a preferred embodiment of the Formula I, **R₃** is an alkenyl chain having 34 carbon atoms, **R₄** is -OCH₃ and **R₅** is -OCH₃.

In a preferred embodiment, the Formula I comprises a lipid chain C34:1 (preferably with a double bond between the carbon 10 and 11) and the carbohydrate moieties are respectively 3-O-Me-6-deoxytalose and 3,4-di-O-Me-α-L-Rhap.

Particularly, the method of the invention for the diagnosis of NTM infections can be applied in the following situations:
- For the diagnosis of diseases directly caused by NTM infections: For example, pulmonary infections, lymphadenitis and skin and soft tissue infections. Particularly, the method of the invention can be used for the identification of NTM infections in the following situations: (i) Colonization/infection of the airway respiratory tract by NTM in children diagnosed with cystic fibrosis and (ii) NTM infections in patients suffering from respiratory chronic diseases (i.e. COPD and cystic fibrosis), and immunosuppressed adults. As explained in the background of the invention, it is important to consider that the identification of diagnostic methods helping in the distinction between colonization and infection would be very relevant, particularly in the case of patients suffering from respiratory chronic diseases. In these situations the method of the invention will help in the diagnosis of NTM because a positive result will reinforce the role of infection and a negative result will reinforce the role of colonization.
- For the differential diagnosis between NTM and TB infection in children with lymphadenopathy when there is a suspicion of active TB. The treatment of the lymphadenopathy in children requires the extraction of the lymphadenopathy by surgery, and also the prescription of specific treatment, which is different if the lymphadenopathy is due to NTM infection [mainly *M*. *avium*] or a *M*. *tuberculosis* infection. The method of the invention will help to rapidly differentiate between NTM and TB, in order to administrate the correct treatment to the patient.
- For the diagnosis of NTM infections in situations where the NTM immune response confuses TST results in both, human and animal species. The method of the invention aimed at identifying the sensitization with NTM will reduce the number of unnecessary LTBI treatments and the cost of the management of these cases and the consequent social alarm. A positive result will support the idea of a NTM infection instead of LTBI infection.

Particularly, *M. avium* non-serovar glycopeptidolipid (nsGPLs) is used as unique NTM antigens, which are totally specific of NTM, as candidates to create a test for the diagnosis or identification of NTM infections. The method of the invention is based on the detection of cytokines produced by blood T-cells after NTM antigen stimulation. For this purpose, nsGPLs were extracted and further analyzed using thin layer chromatography (TLC) and gas chromatography-mass spectrometry (GC-MS) to confirm their purity.

The present invention shows that nsGPLs, in particular *M*. *avium* nsGPLs, are valid antigens to detect immune response against any NTM species.

The nsGPLs, in particular *M*. *avium* nsGPLs, described in the invention are capable of acting as stimulating antigen, which in turn are capable of diagnosing the NTM infections.

This opens the attractive possibility to create a unique test for NTM diagnostics and for evaluating the role of NTM in LTBI diagnosis. The diagnostic method of the invention will help to prescribe adequate treatments. It will also reduce unnecessary therapies with the consequent benefits preventing antibiotic resistance, reducing unnecessary prophylaxis for LTBI, and also reducing discomfort in the patient that have to receive unnecessary or inadequate therapy.

Thus the first embodiment of the present invention as defined in the appended claims refers to the use of *M*. *avium* non-serovar specific GLP antigens of Formula (I) (see above), for the *in vitro* diagnosis or detection of infections caused not only by *M*. *avium* but caused by any NTM. Disclosed, but not part of the invention, is the use of a non-serovar specific GLP antigen, or an antigen comprising such nsGPL, obtained from a bacteria selected from any of the group consisting of: *Mycobacterium abcessus, Mycobacterium chimaera, Mycobacterium fortuitum, Mycobacterium intracellulare, Mycobacterium malmoense, Mycobacterium xenopi* or *Mycobacterium smegmatis.*

Also disclosed, but not part of the invention, is the use of an antigen of Formula II, or an antigen comprising the chemical structure of formula II, or any isomer thereof.

The second embodiment of the present invention as defined by the appended claims refers to the use of *M*. *avium* non-serovar specific GLP antigens of Formula (I) (see above), for *in vitro* differentiating between infections caused not only by *M. avium* but caused by any NTM, from latent tuberculosis infection. Disclosed, but not part of the invention, is the use of a non-serovar specific GLP antigen, or an antigen comprising such nsGPL, obtained from a bacterium selected from any of the group consisting of: *Mycobacterium abcessus, Mycobacterium chimaera, Mycobacterium fortuitum, Mycobacterium intracellulare, Mycobacterium malmoense, Mycobacterium xenopi* or *Mycobacterium smegmatis.* Also disclosed, but not part of the invention, is the use of an antigen of Formula II, or an antigen comprising the chemical structure of formula II, or any isomer thereof.

The third embodiment of the present invention as defined by the appended claims refers to the use of *M*. *avium* non-serovar specific GLP antigens of Formula (see above), for deciding whether to administer a medical treatment to a subject suspected of suffering from an infection caused not only by *M*. *avium* but caused by any NTM. Disclosed, but not part of the invention, is the use of a non-serovar specific GLP antigen, or an antigen comprising such nsGPL, obtained from a bacteria selected from any of the group consisting of: *Mycobacterium abcessus, Mycobacterium chimaera, Mycobacterium fortuitum, Mycobacterium intracellulare, Mycobacterium malmoense, Mycobacterium xenopi* or *Mycobacterium smegmatis.* Also disclosed, but not part of the invention, is the use of an antigen of Formula II, or an antigen comprising the chemical structure of formula II, or any isomer thereof.

The fourth embodiment of the present invention as defined by the appended claims refers to an *in vitro* method for detecting or diagnosing infections caused not only by *M. avium* but caused by any NTM, said method comprising: a) incubating a biological sample obtained from the subject with any of the antigens mentioned in any of embodiments one to three above, i.e. *M. avium* non-serovar specific GLP antigens of Formula (I), b) quantifying the secreted level of at least one cytokine, c) wherein if the cytokine level quantified in step (b) is higher than the reference control level, it is indicative that the subject is suffering from an infection caused not only by *M. avium* but caused by any NTM.

The fifth embodiment of the present invention as defined by the appended claims refers to an *in vitro* method for differentiating between infections caused not only by *M. avium* but caused by any NTM from latent tuberculosis infection, said method comprising: a) Incubating a biological sample obtained from the subject with any of the antigens mentioned in any of embodiments one to three above, i.e. *M.avium* non-serovar specific GLP antigen of Formula (I), b) quantifying the secreted level of at least one cytokine, c) wherein if the cytokine level quantified in step (b) is higher than the reference control level, it is indicative that the subject is suffering from an infection caused not only by *M. avium* but caused by any NTM, and a latent tuberculosis infection can be discarded when conventional IGRAs test is negative.

The sixth embodiment of the present invention as defined by the appended claims refers to an *in vitro* method for deciding whether to administer a medical treatment to a subject suspected of suffering from an infection caused not only by *M*. *avium* but caused by any NTM, said method comprising: a) Incubating a biological sample obtained from the subject with any of the antigens mentioned in any of embodiments one to three above, i.e. *M.avium* non-serovar specific GLP antigen of Formula (I) b) quantifying the secreted level of at least one cytokine, and c) wherein if the cytokine level quantified in step (b) is higher than the reference control level, it is indicative that the subject is suffering from an infection caused not only by *M*. *avium* but caused by any NTM and a medical treatment directed to NTM could be applied.

In a preferred embodiment, the NTM infection is identified in subjects suffering from: lymphadenitis, skin and soft tissue infection, chronic pulmonary diseases, disseminated disease in immune compromised patients.

Disclosed, but not part of the invention, is the aspect that , the NTM infection is caused by a mycobacteria selected the group comprising: *Mycobacterium avium, Mycobacterium intracellulare, Mycobacterium scrofulaceum, Mycobacterium abscessus, Mycobacterium chelonae, Mycobacterium chimaera Mycobacterium smegmatis, Mycobacterium peregrinum, Mycobacterium simiae, Mycobacterium fortuitum, Mycobacterium marinum, Mycobacterium paratuberculosis, Mycobacterium farcinogenes, Mycobacterium lepraemurium, Mycobacterium porcinum* and *Mycobacterium senegalens,* among others. More particularly, by any of the following bacteria selected from the group consisting of: *Mycobacterium abcessus, Mycobacterium chimaera, Mycobacterium fortuitum, Mycobacterium intracellulare, Mycobacterium malmoense, Mycobacterium xenopi* or *Mycobacterium smegmatis.* In particular, such nsGPLs are capable of detecting infections caused by any of the following bacterial species selected from the list consisting of: *Mycobacterium abcessus, Mycobacterium chimaera, Mycobacterium fortuitum, Mycobacterium intracellulare, Mycobacterium malmoense, Mycobacterium xenopi, Mycobacterium chelonae, Mycobacterium farcinogenes, Mycobacterium Habana, Mycobacterium hominis, Mycobacterium paratuberculosis, Mycobacterium peregrinun, Mycobacterium porcinum, Mycobacterium senegalense, Mycobacterium smegmatis, Mycobacterium scrofulaceum* and *Mycobacterium simiae;* still more particularly by any of the following bacterial species selected from the list consisting of: *Mycobacterium abcessus, Mycobacterium avium, Mycobacterium chimaera, Mycobacterium fortuitum, Mycobacterium intracellulare, Mycobacterium malmoense* and *Mycobacterium xenopi.*

In a preferred embodiment, the cytokine or combination of cytokines are selected from the group comprising: IFN-gamma, TNF-alpha, IP-10, IL-10, IL-1RA, GM-CSF, IL-13, IL-17, IL-5, IL-2, IL-22 and IL-32.

in a preferred embodiment, the biological sample is selected from: whole blood, saliva, bronchoalveolar fluid, cerebrospinal fluid, purified peripheral blood mononuclear cells (PBMCs) or biopsies.

The subject is a human or an animal. On the other hand, the methods used for quantifying the secretion of cytokines in a biological sample are well known in the art. For example, any immunological method such as but not limited to ELISA, multiplex strategies, ELISPOT, immunochromatography techniques, proteomic methods, Western blotting, FACS, or radioimmunoassays may be applicable to the present invention. Typically said methods comprise contacting the biological sample with a binding partner capable of selectively interacting with the biomarkers present in the biological sample. The binding partner may be an antibody that may be polyclonal or monoclonal, preferably monoclonal. In another embodiment, the binding partner may be an aptamer. Polyclonal antibodies or a fragment thereof can be raised according to known methods by administering the appropriate antigen or epitope to a host animal selected, e.g., from pigs, cows, horses, rabbits, goats, sheep, and mice, among others. Various adjuvants known in the art can be used to enhance antibody production. Although antibodies useful in practicing the invention can be polyclonal, monoclonal antibodies are preferred. Monoclonal antibodies or a fragment thereof can be prepared and isolated using any technique that provides for the production of antibody molecules by continuous cell lines in culture. Techniques for production and isolation include but are not limited to the hybridoma technique originally described by Kohler and Milstein (1975); the human B-cell hybridoma technique (Cote et al., 1983); and the EBV-hybridoma technique (Cole et al. 1985). Alternatively, techniques described for the production of single chain antibodies (see e.g. U.S. Pat. No. 4,946,778) can be adapted to produce anti-cytokine, single chain antibodies. Antibodies useful in practicing the present invention also include anti-cytokine fragments including but not limited to F(ab')2 fragments, which can be generated by pepsin digestion of an intact antibody molecule, and Fab fragments, which can be generated by reducing the disulfide bridges of the F(ab')2 fragments. Alternatively, Fab and/or scFv expression libraries can be constructed to allow rapid identification of fragments having the desired specificity to cytokine. For example, phage display of antibodies may be used. In such a method, single-chain Fv (scFv) or Fab fragments are expressed on the surface of a suitable bacteriophage, e.g., M13. Briefly, spleen cells of a suitable host, e. g., mouse, that has been immunized with a protein are removed. The coding regions of the VL and VH chains are obtained from those cells that are producing the desired antibody against the protein. These coding regions are then fused to a terminus of a phage sequence. Once the phage is inserted into a suitable carrier, e.g., bacteria, the phage displays the antibody fragment. Phage display of antibodies may also be provided by combinatorial methods known to those skilled in the art. Antibody fragments displayed by a phage may then be used as part of an immunoassay. In another embodiment, the binding partner may be an aptamer. Aptamers are a class of molecule that represents an alternative to antibodies in term of molecular recognition. Aptamers are oligonucleotide or oligopeptide sequences with the capacity to recognize virtually any class of target molecules with high affinity and specificity. Such ligands may be isolated through Systematic Evolution of Ligands by Exponential enrichment (SELEX) of a random sequence library, as described in Tuerk C. 1997. The random sequence library is obtainable by combinatorial chemical synthesis of DNA. In this library, each member is a linear oligomer, eventually chemically modified, of a unique sequence. Possible modifications, uses and advantages of this class of molecules have been reviewed in Jayasena S.D., 1999. Peptide aptamers consist of conformationally constrained antibody variable regions displayed by a platform protein, such as E. coli Thioredoxin A, that are selected from combinatorial libraries by two hybrid methods (Colas et al, 1996). The binding partners such as antibodies or aptamers, may be labelled with a detectable molecule or substance, such as a fluorescent molecule, a radioactive molecule or any others labels known in the art. Labels are known in the art that generally provide (either directly or indirectly) a signal. As used herein, the term "labelled", with regard to the antibody, is intended to encompass direct labelling of the antibody or aptamer by coupling (i.e., physically linking) a detectable substance, such as a radioactive agent or a fluorophore (e.g. fluorescein isothiocyanate (FITC) or phycoerythrin (PE) or Indocyanine (Cy5)) to the antibody or aptamer, as well as indirect labelling of the probe or antibody by reactivity with a detectable substance. An antibody or aptamer may be labelled with a radioactive molecule by any method known in the art. For example, radioactive molecules include but are not limited radioactive atom for scintigraphic studies such as 1123, 1124, In111, Re 186, Re188. The aforementioned assays generally involve the binding of the binding partner (ie. antibody or aptamer) to a solid support. Solid supports which can be used in the practice of the invention include substrates such as nitrocellulose (e. g., in membrane or microtiter well form); polyvinylchloride (e. g., sheets or microtiter wells); polystyrene latex (e.g., beads or microtiter plates); polyvinylidine fluoride; diazotized paper; nylon membranes; activated beads, magnetically responsive beads, plastic or glass (e.g. blood collection tubes). In a particular embodiment, an ELISA method can be used, wherein the wells of a microtiter plate are coated with a set of antibodies which recognize said cytokine(s). A biological sample containing or suspected of containing said cytokine(s) is then added to the coated wells. After a period of incubation sufficient to allow the formation of antibody-antigen complexes, the plate(s) can be washed to remove unbound moieties and a detectably labelled secondary binding molecule added. The secondary binding molecule is allowed to react with any captured sample marker protein, the plate washed and the presence of the secondary binding molecule detected using methods well known in the art. In one embodiment, an Enzyme-linked immunospot (ELISpot) method may be used. For the ELISPOT method detection, the biological sample is sent to the laboratory and then the PBMCS are isolated and incubated with the antigens there. Typically, the biological sample is transferred to a plate which has been coated with the desired anti-cytokine capture antibodies. Revelation is carried out with biotinylated secondary Abs and standard colorimetric or fluorometric detection methods such as streptavidin-alkaline phosphatase and NBT-BCIP and the spots counted. In one embodiment, when multi-cytokine secretion quantification is required, use of beads bearing binding partners of interest may be preferred. In a particular embodiment, the bead may be a cytometric bead for use in flow cytometry. Such beads may for example correspond to BD^{™} Cytometric Beads commercialized by BD Biosciences (San Jose, California). Typically, cytometric beads may be suitable for preparing a multiplexed bead assay. A multiplexed bead assay, such as, for example, the BD^{™} Cytometric Bead Array, is a series of spectrally discrete beads that can be used to capture and quantify soluble antigens. Typically, beads are labelled with one or more spectrally distinct fluorescent dyes, and detection is carried out using a multiplicity of photodetectors, one for each distinct dye to be detected. A number of methods of making and using sets of distinguishable beads have been described in the literature. These include beads distinguishable by size, wherein each size bead is coated with a different target-specific antibody (see e.g. Fulwyler and McHugh, 1990, Methods in Cell Biology 33:613-629), beads with two or more fluorescent dyes at varying concentrations, wherein the beads are identified by the levels of fluorescence dyes (see e.g. European Patent No. 0 126,450), and beads distinguishably labelled with two different dyes, wherein the beads are identified by separately measuring the fluorescence intensity of each of the dyes (see e.g. U.S. patent Nos. 4,499,052 and 4,717,655). Both one-dimensional and two-dimensional arrays for the simultaneous analysis of multiple antigens by flow cytometry are available commercially. Examples of one-dimensional arrays of singly dyed beads distinguishable by the level of fluorescence intensity include the BD^{™} Cytometric Bead Array (CBA) (BD Biosciences, San Jose, Calif.) and Cyto-Plex^{™} Flow Cytometry microspheres (Duke Scientific, Palo Alto, Calif). An example of a two-dimensional array of beads distinguishable by a combination of fluorescence intensity (five levels) and size (two sizes) is the QuantumPlex(TM) microspheres (Bangs Laboratories, Fisher, Ind.). An example of a two-dimensional array of doubly-dyed beads distinguishable by the levels of fluorescence of each of the two dyes is described in Fulton et al. (1997, Clinical Chemistry 43(9):1749-1756). The beads may be labelled with any fluorescent compound known in the art such as e.g. FITC (FL1), PE (FL2), fluorophores for use in the blue laser (e.g. PerCP, PE-Cy7, PE-Cy5, FL3 and APC or Cy5, FL4), fluorophores for use in the red, violet or UV laser (e.g. Pacific blue, pacific orange). In another particular embodiment, bead is a magnetic bead for use in magnetic separation. Magnetic beads are known to those of skill in the art. Typically, the magnetic bead is preferably made of a magnetic material selected from the group consisting of metals (e.g. iron, cobalt and nickel), an alloy thereof and an oxide thereof. In another particular embodiment, bead is bead that is dyed and magnetized.

In one embodiment, for the in-Tube version of the technology, the incubation of the biological sample with the mycobacteria antigens is performed at the point of care locations such as physicians' offices, clinics, or outpatient facilities. Once incubation is complete, the requirement for fresh and active cells no longer exists. Cytokines are stable and, thus, the biological sample can be stored, frozen or shipped without special conditions. Accordingly, in one embodiment, the biological sample is collected in suitable container (e.g. collection tube) containing the mycobacteria antigen or a plurality of mycobacteria antigens. The incubation step may be from 5 to 48 hours, more preferably 5 to 36 hours and even more preferably 12 to 24 hours or a time period in between. Thus in one embodiment the incubation time is 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, 24 hours, 26 hours, 30 hours, 36 hours, 42 hours, or 48 hours.

Although, as demonstrated in the examples, the inventors have been able to identify not only infections caused by *M. avium* but also infections caused by any NTM by using precisely *M*. *avium* nsGPLs antigens, it is herein again noted that the present examples also offer scientific evidences to prove that the use of other different nsGPLs pertaining to different NTM could be used for detecting infections caused by any NTM. In fact, **Example 6 and example 8 offer** clear evidence that *M*. *smegmatis* nsGPLs antigens, can also be used to identify non only infections caused by *M. smegmatis* but also infections caused by any NTM. For the purpose of the present invention:
- The term "*M*. *avium nsGPL antigens"* refers to GPLs present in the cell wall wherein the oligosaccharide portion which defines the different serovars of the different NTM species is not present. In a preferred embodiment, the "*M*. *avium nsGPLs"* comprises the Formula I.
- The term "comprising" it is meant including, but not limited to, whatever follows the word "comprising". Thus, use of the term "comprising" indicates that the listed elements are required or mandatory, but that other elements are optional and may or may not be present.
- By "consisting of" is meant including, and limited to, whatever follows the phrase "consisting of". Thus, the phrase "consisting of" indicates that the listed elements are required or mandatory, and that no other elements may be present.
- The term "reference control level", when referring to the level of the biomarkers (cytokines) described in the present invention, refers to the level observed in patients, which are not infected with NTM. The patient is likely to be infected with NTM with a given sensitivity and specificity if the levels of the biomarker (cytokines) in the patient are above said "reference control level". A "reference" value can be a threshold value or a cut-off value. Typically, a "threshold value" or "cut-off value" can be determined experimentally, empirically, or theoretically. A threshold value can also be arbitrarily selected based upon the existing experimental and/or clinical conditions, as would be recognized by a person of ordinary skilled in the art. The threshold value has to be determined in order to obtain the optimal sensitivity and specificity according to the function of the test and the benefit/risk balance (clinical consequences of false positive and false negative). Preferably, the person skilled in the art may compare the cytokine secretion levels (or scores) obtained according to the method of the invention with a defined threshold value. The threshold value may be derived from the cytokine secretion level (or score) determined in a control sample derived from one or more subjects who are substantially healthy (i.e. having no NTM). The threshold value may also be derived from cytokine secretion level (or score) determined in a control sample derived from one or more subjects who suffers from NTM. Furthermore, retrospective measurement of the cytokine secretion levels (or scores) in properly banked historical subject samples may be used in establishing these threshold values. Typically, the optimal sensitivity and specificity (and so the threshold value) can be determined using a Receiver Operating Characteristic (ROC) curve based on experimental data. For example, after determining the levels of the cytokines in a group of reference, one can use algorithmic analysis for the statistic treatment of the measured concentrations of biomarkers in biological samples to be tested, and thus obtain a classification standard having significance for sample classification. The full name of ROC curve is receiver operator characteristic curve, which is also known as receiver operation characteristic curve. It is mainly used for clinical biochemical diagnostic tests. ROC curve is a comprehensive indicator that reflects the continuous variables of true positive rate (sensitivity) and false positive rate (1-speciftcity). It reveals the relationship between sensitivity and specificity with the image composition method. A series of different cut-off values (thresholds or critical values, boundary values between normal and abnormal results of diagnostic test) are set as continuous variables to calculate a series of sensitivity and specificity values. Then sensitivity is used as the vertical coordinate and specificity is used as the horizontal coordinate to draw a curve. The higher the area under the curve (AUC), the higher the accuracy of diagnosis. On the ROC curve, the point closest to the far upper left of the coordinate diagram is a critical point having both high sensitivity and high specificity values. The AUC value of the ROC curve is between 1.0 and 0.5. When AUC>0.5, the diagnostic result gets better and better as AUC approaches 1. When AUC is between 0.5 and 0.7, the accuracy is low. When AUC is between 0.7 and 0.9, the accuracy is moderate. When AUC is higher than 0.9, the accuracy is quite high. This algorithmic method is preferably done with a computer. Existing software or systems in the art may be used for the drawing of the ROC curve, such as: MedCalc 9.2.0.1 medical statistical software, SPSS 9.0.
- The term "positive result", as used herein, refers to the situation when the levels of the biomarker (cytokines) measured in the biological sample obtained from the patient are above the "reference control level". In this case, the patient is likely to be infected with NTM species.
- The term "negative result" as used herein refers to the situation when the levels of the biomarker (cytokines) measured in the biological sample obtained from the patient are equal or below the "reference control level". In this case, the patient is not likely to be infected with NTM species.
- The term "biological sample" as used herein refers to whole blood, saliva, bronchoalveolar fluids, cerebrospinal fluids, or purified peripheral blood mononuclear cells (PBMC), or any of other biological fluids or tissues, in condition that they contain leucocytes, and especially T-cells. Biological fluids have been isolated from the subject and collected in tubes or other containers containing an appropriate anti-coagulant when required (e.g., lithium heparin or sodium citrate). For example, the crude whole blood specimen is unfractionated whole blood collected with appropriate anti-coagulant (e.g. EDTA, heparin or sodium citrate). It contains plasma and blood cells (red blood cells, white blood cells). It may be a freshly isolated blood sample (<32h) or a blood sample, which has been obtained previously and kept frozen until use. T-Cell *Xtend* reagent (Oxford Immunotec, Abingdon, UK) could be added in blood samples processed >32h post-venipuncture. T-Cell *Xtend* ensures the functionality of T-cells when whole blood is stored overnight. Typically, the biological sample (e.g. blood sample) comprises PBMCs, including T cells such as CD4 T cells, CD8 T cells, B cells, gamma-delta T cells but also monocytes, macrophages and NK cells.
- The term "isomer" refers to a molecule with the same molecular formula as any of the compounds derived from the Formula I, but with a different chemical structure. Isomers contain the same number of atoms of each element, but have different arrangements of their atoms. This includes the two main types of isomers: structural isomerism (which includes molecules with the same molecular formula having different bonding patterns and atomic organization) and stereoisomerism (which includes molecules with the same molecular formula and sequence of bonded atoms, but differs in the three-dimensional orientations of their atoms in space).

### Brief Description of the Figures

**Figure 1****.** Scheme of the protocol followed in the method of the invention. This protocol is based on the antigen stimulation of the biological samples, and the cytokine detection by ELISPOT. However, it also works when antigens are fixed directly into the blood collection tubes (In tube technology), and the cytokines detection performed by ELISA.

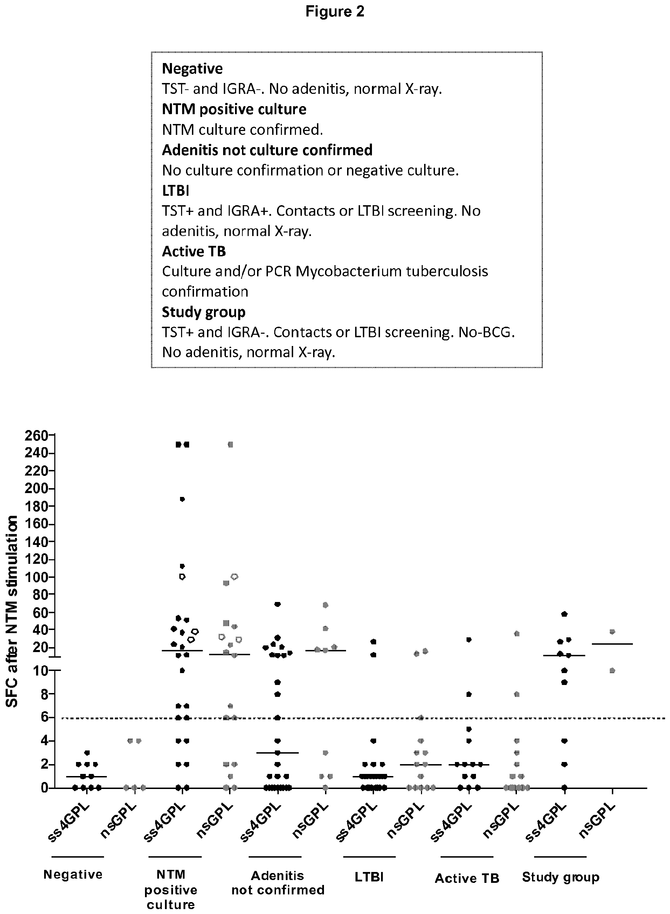

**Figure 2****.** Graph with the spot-forming cells (SFC) obtained after stimulation with M. *avium* serovar specific 4 GPLs (ss4GPLs; black dots) and nonspecific GPLs (nsGPLs; grey dots) per patient group and using the ELISPOT technology. The total number of patients analyzed by ss4GPLs and nsGPLs were n=109 and n=63, respectively. Open symbols represent patients with cystic fibrosis. Indeterminate results were excluded. The dotted line represented the positivity cut-off (SCF= 6). nsGPLs in the figure refer to *M*. *avium* ns4GPLs.

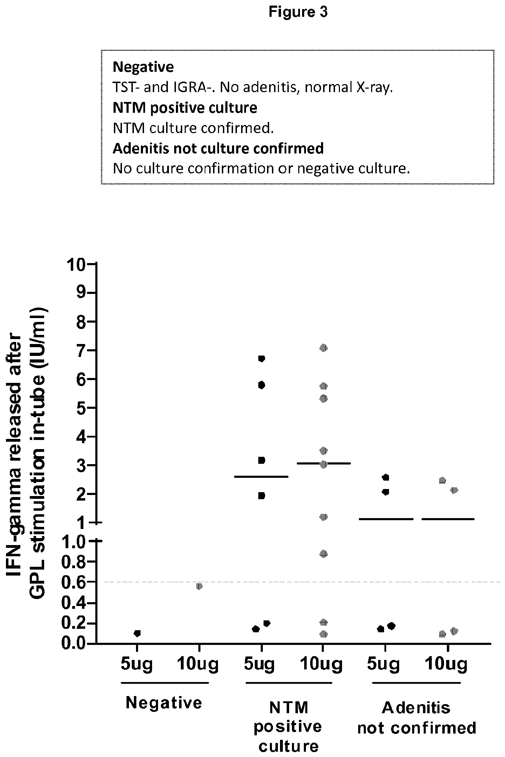

**Figure 3****.** Graph showing the IFN-gamma released (IU/ml) per patient group using the in-tube stimulation technology. Whole blood was stimulated with two different concentrations using 5µg (black dots; n=11) and 10 µg (grey dots; n=14) of *M*. *avium* nsGPLs. Indeterminate results were excluded. Dotted line represented the positivity cut-off (IU/ml=0.6).

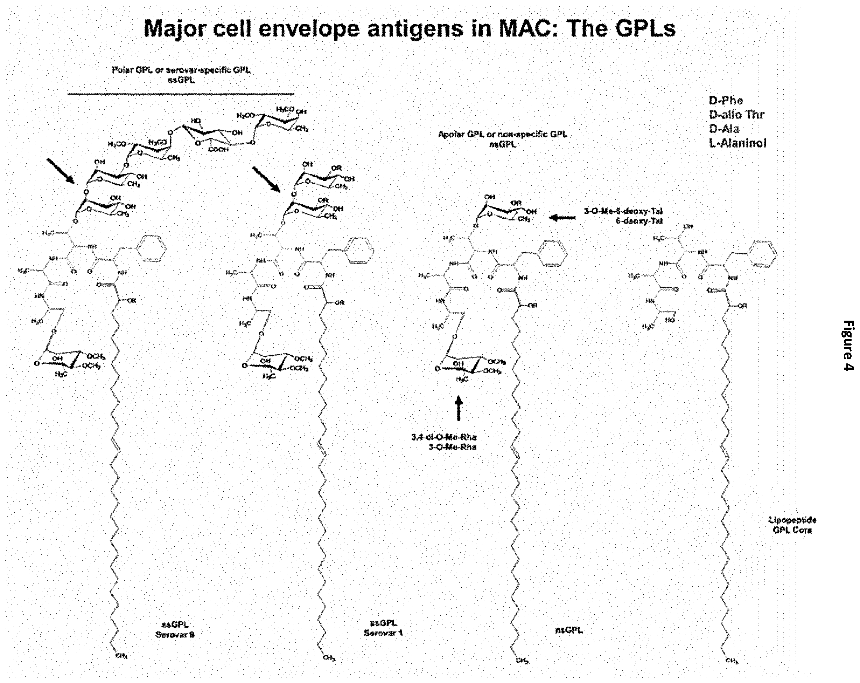

**Figure 4****.** Scheme showing the chemical structure of lipopeptide core, nsGPL and different serovar-specific GPLs in *M*. *avium.* The oligosaccharide portion defines the different serovars.

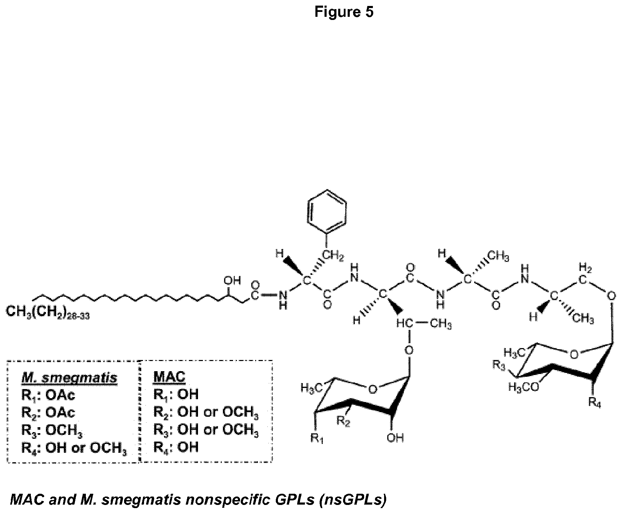

**Figure 5****.** The nonspecific GPLs (nsGPLs) of *M*. *avium* and *M*. *smegmatis.* These GPLs share the same di-glycosylated N-linked fatty acyl tripeptide-amino-alcohol core. However, there are slight differences in the modification of the α-I-rhamnose (rhamnose) and 6-deoxy-α-I-talose (6-deoxytalose). In the case of *M*. *avium,* the rhamnose is either 3-O-methylated or 3,4-di-O-methylated, and the 6-deoxytalose is 3-O-methylated or nonmethylated. In the case of *M*. *smegmatis,* its nsGPLs contain a 3,4-di-O-methyl rhamnose or a 2,3,4-tri-O-methyl rhamnose, and the 6-deoxytalose is usually 3,4-di-O-acetylated. MAC strains may also have acetyl groups at various undefined locations on the GPL depending on the strain of mycobacteria.

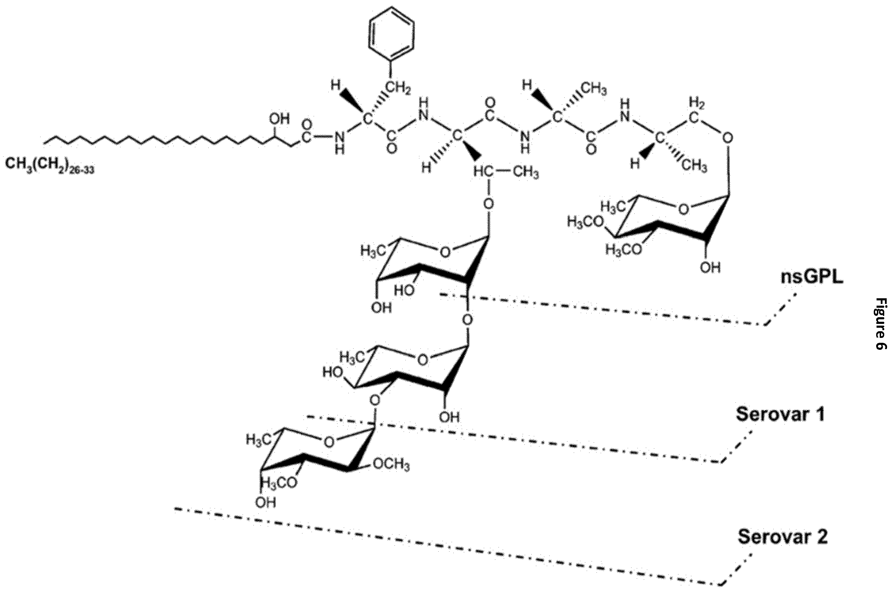

**Figure 6****.** MAC strains produce polar, serovar-specific GPLs (ssGPLs). ssGPLs contain an α-L-rhamnose that is always 3,4-di-O-methylated, and the nonmethylated 6-deoxy-α-l-talose is extended with additional carbohydrate moieties. Different ssGPLs possess different oligosaccharide appendages. For example, the serovar 1 GPL contains an *α*-I-rhamnose-(1→ 2)-6-deoxy- *α*-I-talose linked to the *allo*-threonine of the lipopeptide core, and the serovar 2 GPL contains a 2,3-di-O-methyl-*α*-l-fucose-(1→3) linked to the rhamnose of the serovar 1 GPL.
**Figure 7****.** Graph showing the spot-forming cells (SFC) obtained after stimulation with *M. avium* nonspecific GPLs (ns4GPLs) using the ELISPOT technology on patients with respiratory chronic diseases with a NTM isolation; and classified as: (i) colonized, (ii) infected or (iii) with previous infection. Indeterminate results were excluded. The dotted line represented the positivity cut-off (SCF= 6). **p<0.001

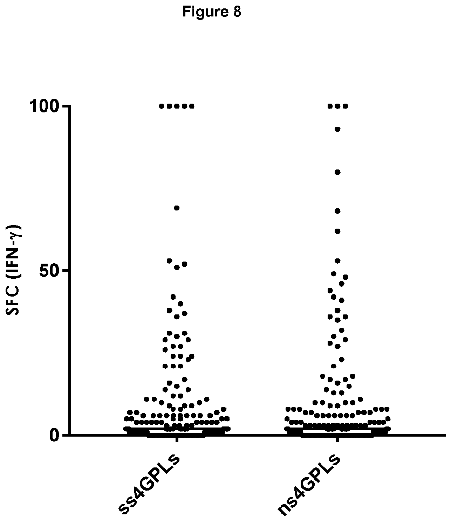

**Figure 8****.** Graph comparing the spot-forming cells (SFC) obtained in the overall population after stimulation with ss4GPLs and ns4GPLs. There were no significant differences among SFCs when stimulating with any of the two GPLs antigens (p>0.05).

### Detailed description of the invention

The present invention is also illustrated by the Examples shown below. They should not be interpreted as a limitation of the scope of the invention, which is defined by the appended claims.

### EXAMPLES

### Glossary of terms

- nsGPL: non-serovar specific Glycopeptidelipid
- ssGPL: serovar specific Glycopeptidelipid
- ss4GPL: serovar specific 4 Glycopeptidelipid from M.avium
- ns4: non-serovar specific GPL obtained from a ss4GPL M.avium strain
- ns8: non-serovar specific GPL obtained from a ss8GPL M.avium strain
- MAC: Mycobacterium avium complex
- MAI: Mycobacterium avium-intracellulare
- HC: Healthy controls. Individuals with negative TST and IGRAs, no adenopathies and normal X-ray
- LTBI: Individuals diagnosed of Latent TB Infection by TST and IGRAs

### Example 1. Method for obtaining the nsGPLs :

### Process for obtaining total GPLs (nsGPLs + ssGPLs):

*M. avium* complex colonies were scraped from the surface of Middlebrook 7H11 agar plates containing oleic acid-albumin-dextrose-catalase (OADC) enrichment, and were transferred aseptically to sterile screw-capped glass culture tubes (20 x 120 mm) containing 5 ml of methanol (CH₃OH) followed by the addition of 10 ml of chloroform (CHCl₃) for a final ratio of CHCl₃-CH₃OH (2:1 v/v) and incubated at 37°C overnight. After this incubation period, samples were centrifuged for 5 min at 11,500 x g at 4°C to obtain supernatants (organic extractions containing total lipids). Then, total lipids were treated with mild alkali (an equal volume of 0.2 N sodium hydroxide (NaOH) in CH₃OH at 37 °C for 35 min) followed by neutralization using 100 microliters of glacial acetic acid (2 to 3 drops, check pH to be 7.0). Mild alkali hydrolysis destroys alkalilabile non-specific acylglycerols, thus serving as a purification step for the alkaline resistant GPLs. The alkali-treated lipids were further partitioned between CHCl₃-CH₃OH (2:1 v/v) and H₂O at a ratio of 1:1 (v/v) to remove generated sodium acetate salts. This 'washing' step was repeated twice. The final chloroform layer (bottom phase) containing the total GPLs fraction was transferred to a pre-weight tube and dried down under nitrogen to avoid lipid oxidation. Dried samples were kept at -20°C under nitrogen until further use. This allows obtaining total GPLs (nsGPLs + ssGPLs).

### Process for purifying ssGPLs from nsGPLs:

Total GPLs extracts were further processed. SEPPAK Classic Silica long body cartridges (Waters Corporation, Milford, MA, USA) were fitted with 5 ml glass syringes, which acted as reservoirs for the eluting solvents. The SEPPAK cartridges were equilibrated with CHCl₃ (5 ml x 3 times) followed by the application of total GPLs dissolved in 0.5 ml CHC1₃. The cartridge was then irrigated with 3 ml of CHCl₃ followed by 2, 10, 25, 35 and 50% CH₃OH in CHCl₃. Finally, the cartridge was washed several times with 5ml CH₃OH. The nsGPLs were removed with CHCl₃ and with 2% CH₃OH in CHCl₃. The total ssGPL population was collected in 20-35% CH₃OH eluate. Fractions were examined for purity by TLC in CHCl₃-CH₃OH-H₂O (60:35 : 8. v/v/v).

For the removal of N-linked acyl chains from GPLs, it is necessary to use the method known as beta-elimination:
Purified nsGPLs or ssGPLs (150 mg) were dissolved in 50% aqueous methanol [using pyrogen-free distilled water (1:1 v/v)] containing 1 N sodium hydroxide (NaOH) and 1 M sodium borohydrate (NaBH4) (a.k.a. sodium tetrahydridoborate or sodium tetrahydroborate) and then heated at 60°C for 24 h, neutralized with acetic acid, and evaporated on a rotary evaporator before being partitioned in CHCl₃-CH₃OH-H₂O (8:4:3, v/v/v). The bottom layer (CHCl₃ layer) was washed twice by adding ½ volume of H₂O, vortex, and the resulting mixture centrifuged for 5 min at 11 500 g (keeping and combining all the water layers from each wash). The final bottom layer (CHCl₃ layer) (containing the resulting beta-lipid was dried down under nitrogen to avoid oxidation and stored under nitrogen at -20°C until further use. The upper phases (aqueous layer) obtained from each wash were combined, concentrated using a speed-ac and desalted using Sephadex G-25 to obtain the reduced oligosaccharide. This technique destroys the peptide into single ammino acids. It is difficult only obtain the glycopeptide. Conversely, it is possible to purify the lipopeptide.

### Example 2. Methodology

Blood is drawn from every patient into two set of tubes: (i) CPT tubes (Becton Dickinson Diagnostics, Franklin Lakes, NJ) for the later isolation of PBMCs, and (ii) blood collection tubes in which the antigens have been previously pre-fixed (see figure 1).

The first approach is based on the enzyme-linked immunospot assay (ELISPOT). Briefly, PBMCs are stimulated with the selected antigens (ss4GPLs and nsGPLs), as well as, positive and reference controls, in a pre-coated well plate. After an overnight incubation, the plate is washed and developed, and the production of IFN-gamma is measured by counting the spot forming cells (SFC).

The second approach is based on the stimulation of whole blood in blood collection tubes in which the antigens have already been fixed. For this procedure the antigens used are ss4GPLs and nsGPLs, as well as a reference and positive control. After an overnight incubation, plasma is harvested, and afterwards the production of IFN-gamma and other cytokines is detected.

### Example 3. Results analysis for each methodology

### Positivity cut-offs for the ELISPOT/In tube-ELISA procedure

In order to establish positivity cut-offs for SFC, we performed a ROC curve analysis. We considered as positive controls those patients with a positive NTM culture and as reference (uninfected) controls, those individuals without NTM or LTBI infection(s). The positivity cut-off was established at five SFC, which gave the greatest specificity (100%) without great sensitivity loss (70,59%). However, based on our previous experience we selected six SFC as the cut-off. Therefore, tested wells are considered positive if they contain at least six SFC more than the reference control well and if this number is at least twice the number of the negative control well.

Regarding the IFN-gamma detection in whole blood by ELISA after in-tube stimulation the cut-off was arbitrary defined as 0,6UI/ml (Figure 3).

Moreover, a result is considered indeterminate in the following three cases: (i) if the response to the antigens is negative (ii) if the number of SFCs in the control positive well is less than 20, and (iii) if the number of SFCs in the reference control is greater than 10.

**Example 4. Percentage of positives obtained after stimulation with ss4GPLs or nsGPLs per patient group using ELISPOT in-house technology.** A total of 109 and 63 patients were stimulated with ss4GPLs and nsGPLs respectively. Patients were classified in six different groups: (i) healthy controls (individuals with negative TST and IGRAs, no adenopathies and normal X-ray). (ii) NTM positive culture (patients with a NTM confirmed culture). (iii) Adenitis not culture confirmed (patients with adenopathies and no NTM culture confirmation/negative culture). (iv) LTBI (individuals with positives TST and IGRAs, coming from contact tracing or LTBI screening studies, without adenopathies and normal X-ray). (v) Active TB (patients with culture and/or PCR *Mycobacterium tuberculosis* confirmation). (vi) Study group (non-BCG vaccinated individuals with a positive TST and a negative IGRA, coming from contact tracing studies or LTBI screenings, without adenopathies and normal X-ray). PBMCs were isolated from each individual and stimulated with ss4GPLs and/or nsGPLs. The presence of sensitized T-cells against the antigens was analyzed by means of an ELISPOT in-house detecting spot-forming cells secreting IFN-gamma.

As shown in **Table 1,** any healthy control showed a positive ELISPOT result after stimulation with both of the antigens. When patients with a positive NTM culture isolation were analyzed, a total of 76.9% (20/26) and 77.8% (14/18) positive results were obtained after ss4GPLs and/or nsGPLs stimulation respectively. For this specific group is detailed the type of mycobacteria isolated. Patients with not culture confirmed adenopathies presented 48.1% (13/27) and 77.8% (7/9) of positive results for ss4GPLs and nsGPLs. The response to ss4GPLs and nsGPLs in LTBI individuals were 8.7% (2/23) and 14.3% (2/14) respectively. Two patients with active TB responded to ss4GPLs (15.4%; 2/13) and nsGPLs (13.3%; 2/15). In addition, the 70% (7/10) and 100% (2/2) of the individuals coming from the study group had a positive response against ss4GPLs and nsGPLs. These results reinforced the hypothesis that non-BCG children, who respond to TST and have a negative IGRA result, could be sensitized to NTMs. Number of spot-forming cells after stimulation with both of the antigens is detailed in **Figure 1** per patient group.

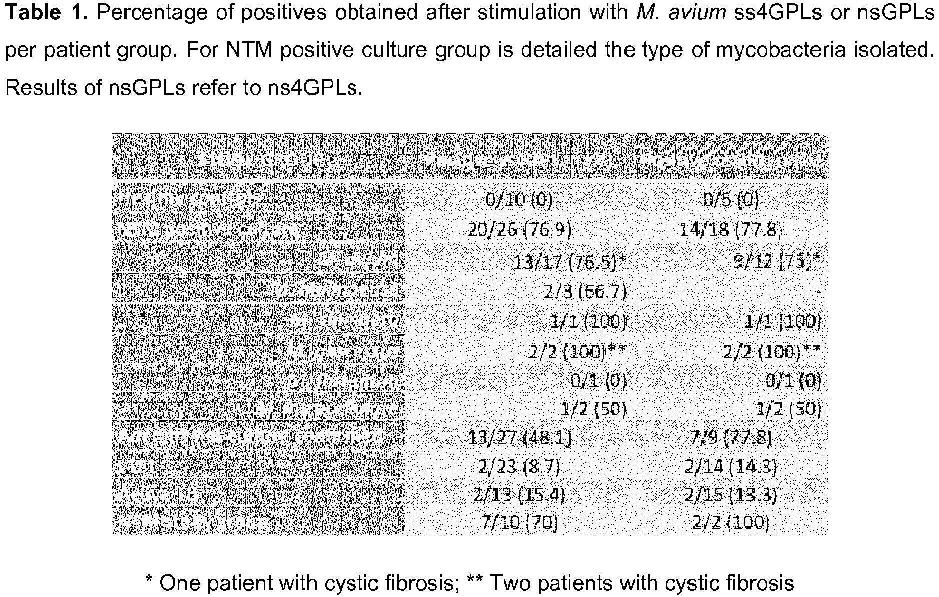

**Example 5. Percentage of positives obtained after stimulation with nsGPLs per patient group using in-tube technology.** A total of 14 patients were stimulated in-tube with 10µg of M. *avium* nsGPLs **(Table 2).** Patients were classified into three groups: (i) healthy controls (individuals with negative TST and IGRAs, no adenopathies and normal X-ray). (ii) NTM positive culture (patients with a NTM confirmed culture). (iii) Adenitis not culture confirmed (patients with adenopathies and no NTM culture confirmation/negative culture). Whole blood was directly incubated in-tube with nsGPLs. The amount of IFN-gamma released (IU/ml) was measured by ELISA. The one healthy individual tested by this technology presented a negative response when stimulated with nsGPLs. When patients with a positive NTM culture isolation and not culture confirmed adenopathies were analyzed, a total of 77.8% (7/9) and 50% (2/4) positive results were obtained respectively. The total amount of IFN-gamma released detailed per patient group after nsGPLs stimulation is represented in **Figure 2****.**

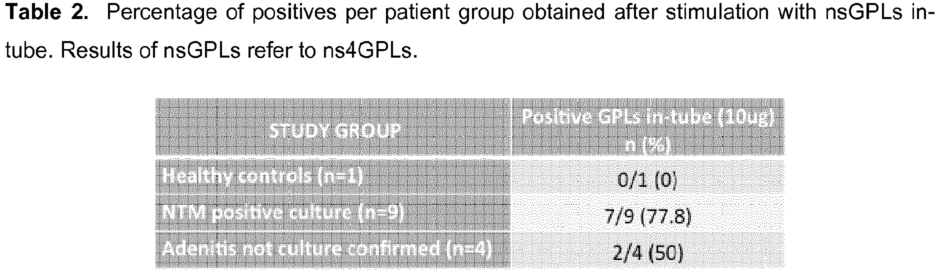

**Example 6. Percentage of positive results obtained after stimulation with nsGPLs of *Mycobacterium smegmatis.*** A total of 22 patients were tested for *Mycobacterium smegmatis* nsGPLs by the ELISPOT in-house technology **(Table 3).** Patients were classified into three groups: (i) healthy controls (individuals with negative TST and IGRAs, no adenopathies and normal X-ray). (II) patients diagnosed of LTBI infection by TST and IGRAs. (iii) NTM positive culture (patients with a NTM confirmed culture). (iv) Adenitis not culture confirmed (patients with adenopathies and no NTM culture confirmation/negative culture). None of the patients belonging to healthy control or LTBI responded to this antigen. Eight from 12 NTM positive culture groups responded to this antigen (66.7%); and only two patients from 8 with not culture confirmed adenopathies had a positive response after stimulation with nsGPLs of M. *smegmatis* (25%; 2/8).

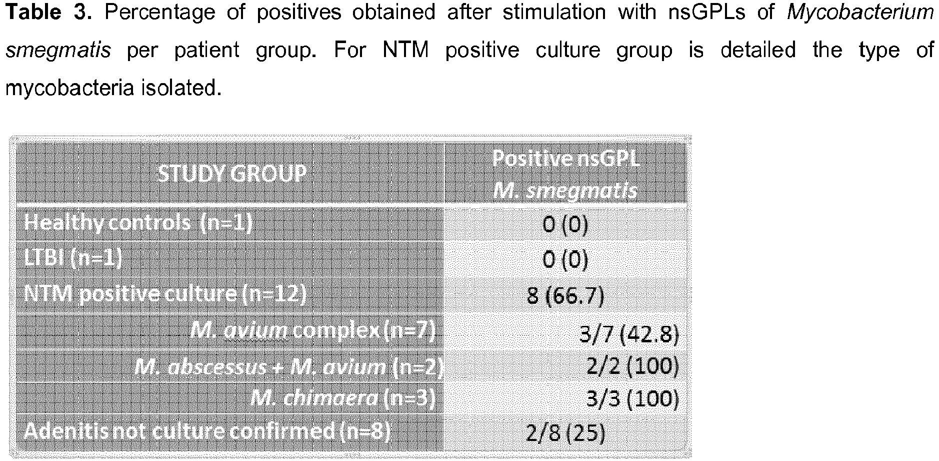

### Example 7. Stimulation with peptidic fraction of the nsGPL.

In order to determine whether or not the peptidic fraction of the nsGPL was itself immunogenic, the inventors of this invention performed a series of experiments in which PBMCs were not only stimulated with the whole molecule of ss4GPLs and nsGPLs, but also with the peptidic fraction. The procedure followed was the same as described in the **Example 2** and illustrated in **Figure 1****.** The peptidic fraction (D-Phe-D-*allo*-Thr-D-Ala-L-Alanol) was synthesized by JPT Innovative Peptide Solutions (JPT Peptide Technologies GmbH - Volmerstrasse 5 - 12489 Berlin). A total of 10 patients (2 healthy controls and 8 patients with a NTM culture confirmed) were tested by ELISPOT with the peptidic fraction of nsGPLs. Such as it can be seen in the **Table 4,** none of the patients included responded to the peptides.

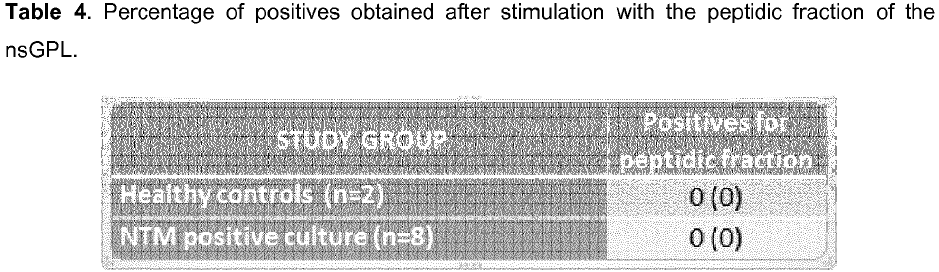

### Example 8. Percentage of positive results obtained after stimulation with ss4GPL, ns4GPL, ns8GPL, total lipid and mixing ss4GPL and ns4GPL of Mycobacterium avium, and nsGPL of Mycobacterium smegmatis.

In order to determine whether or not the immunogenicity of the total lipids, nsGPL obtained from different *M.avium* serovar, or even from other species, the inventors of the present invention performed a series of experiments in which PBMCs were stimulated with the whole molecule of ss4GPLs, and nsGPLs obtained from ss4 and ss8 M. *avium* strains, total lipid, a mix of ss4 and ns4 and nsGPL from *M. smegmatis.* A total of 50 patients were tested for the antigens by the ELISPOT in-house technology (Table 5). Patients were classified into three groups: (i) healthy controls (individuals with negative TST and IGRAs, no adenopathies and normal X-ray), (ii) patients diagnosed with LTBI infection by TST and IGRAs, (iii) NTM positive culture (patients with a NTM confirmed culture), (iv) adenitis not culture confirmed (patients with adenopathies and no NTM culture confirmation/negative culture), (v) Active TB (patients with culture and/or PCR *Mycobacterium tuberculosis* confirmation), (vi) Study group (non-BCG vaccinated individuals with a positive TST and a negative IGRA, coming from contact tracing studies or LTBI screenings, without adenopathies and normal X-ray).

As shown below, the immunogenicity of the antigens tested was very similar, not showing significant differences in the distribution of positive results between the different groups of patients tested (Table 5 and 6). Given the non-significant differences between results detected using *Mycobacterium avium* ss4GPLs and ns4GPLs (Table 5 and 6, Figure 8), results obtained by any of these two GPLs (ss4GPLs or ns4GPLs) are detailed in Table 7.

**Table 5. Percentage of positives obtained after stimulation with ss4GPL, ns4GPL, ns8GPL, total lipid and mixing ss4GPL and ns4GPL of Mycobacterium avium, and nsGPL of Mycobacterium smegmatis per patient group. For NTM positive culture group is detailed the type of mycobacteria isolated.**

| | **Positive (%)** | | | | | |
|---|---|---|---|---|---|---|
| **Samples tested (n=49)** | **ss4** | **ns4** | **ns8** | **Total** | **ss4+ns4 mix** | **M. smeg** |
| **NTM positive (n-12)** | 7^{∗∗∗∗} (58_{.}3) | 8^{∗∗} (66_{.}7) | 6 (50) | 6^{∗∗} (50) | 9^{∗∗∗∗} (75) | 6^{∗∗∗∗} (60) |
| *MAC (n=6)* | 3^{∗∗} (50) | 4* (66.7) | 2 (33.3) | 3** (50) | 3' (50) | 3^{∗∗∗} (50) |
| *M abcessus (n=1)* | 1^{∗}(100) | 1 (100) | 1 (100) | 1 (100) | 1 (100) | 1* (100) |
| *M*. *chimaera (n=1)* | 1 (100) | 1 (100) | 1 (100) | 1 (100) | 1 (100) | 1 (100) |
| *M*. *abcessus* + *M chimaera-intracellulare (n=1)* | - | 1' (100) | 1 (100) | - | 1^{∗} (100) | - |
| *M*. *avium*+ *M abcessus (n=1)* | 1^{∗} (100) | - | - | - | 1^{∗} (100) | - |
| MAI (*2017 treated*) + *M*. *abcessus (9*/*2018. not treated) (n=1)* | 1 (100) | 1(100) | 1 (100) | 1 (100) | 1 (100) | 1 (100) |
| *M*. *celatum (n=1)* | - | - | - | - | 1^{∗} (100) | - |
| **LTBI (n-15)** | 5^{∗} (33.3) | 3 (20) | 6^{∗∗} (40) | 4^{∗} (26.7) | 6^{∗} (40) | 2 (13.3) |
| **Active TB (n=6)** | 0 (0.0) | 0 (0.0) | 0 (0.0) | 1 (16.71 | 1 (16.7) | 0(0.0) |
| **HC (n-9)** | 0 (0.0) | 0 (0.0) | 1 (1 1.1) | 0 (0.0) | 0 (0.0) | 0 (0.0) |
| **Lymphadenopathies not culture confirmed (n=4)** | 1^{∗} (25.0) | 1* (25.0) | 1 (25.0) | 1 (25.0) | 1^{∗} (25.0) | 1^{∗} (25.0) |
| **Study group (n=3)** | 1 (33.3) | 3 (100) | 1 (33.3) | 3 (100) | 2 (66.7) | 0 (0.0) |

| | | | | | | |
|---|---|---|---|---|---|---|
| "The number of ' indicate the number of positive limit values (6-7 SFCi | | | | | | |

**Table 6. Percentage of positives obtained after stimulation with ss4GPL, ns4GPL per patient group. For NTM positive culture group is detailed the type of mycobacteria isolated**

| **ss4GPLs vs nsGPLs (n=181)** | | |
|---|---|---|
| | **Positives (%)** | |
| | **ss4GPLs** | **ns4GPLs** |
| **NTM** + **(n=25) (4 CF)** | | |
| *MAC*^{a} (n=19) | 13^{∗∗∗∗} (68.4) | 15^{∗∗∗} (78.9) |
| *M*. *chimaera (n=1)* | 1 (100.0) | 1 (100.0) |
| *MAC* + M. *abscessus*^{b} *(n*=*4)* | 4^{∗} (100.0) | 3 (75.0) |
| *M*. *abcessus* + M. *chimaera (n=1)* | | - 1^{∗} (100.0) |
| **Ex NTM+ (n=6)** | 1 * (16.7) | 2^{∗∗}(33.3) |
| **LTBI (n=42)** | 14* (33.3) | 10^{∗∗} (23.8) |
| **Active TB (n=29)** | 4 (13.8) | 4^{∗} (13.8) |
| **HC (n-13)** | 0 (0.0) | 0 (0.0) |
| **Lymphadenopathies not culture confirmed (n=8)** | 3^{∗} (37.5) | 3* (37.5) |
| **Lymphadenopathies not NTM (n=1)** | 1 (100.0) | 1 (100.0) |
| **Study group (3 of them clinical LTBI) (n=5)** | 4 (80.0) | 5 (100.0) |
| **HIV (n-52)** | 14 ****** (26.9) | 12 ***(23.1) |

| | | |
|---|---|---|
| *^{a}M. avium, M*. *intracellulare*, *MAI*, *M*. *avium complex*. °2 of them FQ. *The number of * indicate the number of positive results with a limit value. | | |

**Table 7. Percentage of positives obtained after stimulation with any Mycobacterium avium GPL (ss4GPLs or ns4GPLs) per patient group. For NTM positive culture group is detailed the type of mycobacteria isolated**

| **GPLs (n=338)** | |
|---|---|
| | **Positives (%)** |
| **NTM + (n=56) (4 CF)** | 41^{∗∗∗∗∗∗∗} (73.2) |
| MAC^{a} (n=34) (1 ind) | 28^{∗∗∗∗∗∗} (82.3) |
| M. *fortuitum* (n=3) | 0 (0.0) |
| M. *chimaera* (n=4) | 4 (100.0) |
| M. abcessus (n=2) (1 ind = exNTM)) | 1 (50.0) |
| M. xenopi^{b}(n=2) | 1 (50.0) |
| M. malmoense (n=3) | 2 (66.7) |
| MAC + M. abscessus^{c} (n=4) | 3 (75.0) |
| MAC + M. *chimaera* (n=1) | 0 (0.0) |
| M. abcessus + M. *chimaera* (n= 1) | 1^{∗} (100.0) |
| M. abcessus + M. *tuberculosis* (n=1) | 0 (0.0) |
| MAC+ M. kansasii^{c} (n= 1) | 1 * (100.0) |
| **LTBI (n=90)** (2 indeterminates) | 24^{∗∗∗∗∗∗} (26.7) |
| **Active TB (n=69)** (1 indeterminate) | 12*** (17.4) |
| **HC (n=22)** | 0 (0.0) |
| **Lymphadenopathies not culture confirmed (n=32)** | 12* (37.5) |
| **Lymphadenopathies not NTM (n=3)** | 0 (0.0) |
| **Study group (n=13)** (3 of them clinical LTBI) | 10 (76.9) |
| **HIV (n=53)** (3 indeterminates) | 12*** (22.6) |

| | |
|---|---|
| ^{a}M.avium, M. intracellulare, MAI, M. avium complex. ^{b}One with active TB. ^{c}2 of them FQ. ^{∗}The number of ^{∗} indicate the number of positive results with a limit value. | |

### Example 9. Plausability that the evidence presented in example 8 encompasses further NTM bacterial species.

### 1. Mycobacterium chelonae and Mycobacterium smegmatis.

These two NTM species have similarities with the nsGPL structure of *M*. *avium* (Schorey JS and Sweet L. The mycobacteria glycopeptidolipds: structure, function and their role in pathogenesis. Glycobiology 2008, 18:832-841; and Chatterjee D and Khoo KH. The surface glycopeptidolipids of mycobacteria: structures and biological properties. Cellular and Molecular Life Sciences 2001, 58: 2018-2042) In addition, these two NTM species have the same nsGPL structure than *M. xenopi* and *M*. *abcessus,* which both are experimentally detected as demonstrated in example 8. Therefore, we consider plausible that these two NTM species could be detected by our test.

### 2. Mycobacterium farcinogenes, Mycobacterium peregrinun, Mycobacterium porcinum and Mycobacterium senegalense.

These four NTM species have similarities with the nsGPL structure of M. avium (Schorey JS and Sweet L. The mycobacteria glycopeptidolipds: structure, function and their role in pathogenesis. Glycobiology 2008, 18:832-841; and Chatterjee D and Khoo KH. The surface glycopeptidolipids of mycobacteria: structures and biological properties. Cellular and Molecular Life Sciences 2001, 58: 2018-2042). In addition, these four NTM species have the same nsGPL structure than M. fortuitum that is experimentally detected as shown in example 8. Therefore, we consider plausible that these four NTM species could be detected by our test.

### 3. Mycobacterium hominis, Mycobacterium paratuberculosis, and Mycobacterium scrofulaceum.

*M. hominis* and *M. paratuberculosis* are members of the *M.avium* complex. Until recently, *M*. *scrofulaceum* was also considered member of the *M*. *avium* complex. *M. avium* and *M*. *intracellulare* (also members of the *M.avium* complex) are easily detected as shown in example 8. Therefore, we consider plausible that these three NTM species could be detected as defined in the claims.

### 4. Mycobacterium habana and Mycobacterium simiae

**The nsGPLs from both of these two NTM species are identical to the nsGPL structure of** *M. avium* **(2).** Therefore, we consider plausible that these two NTM species could be detected by our test.

## Claims

1. Use of *Mycobacterium avium* non-serovar specific GPL (nonserovar Glycopeptidolipid, nsGPL) antigen of Formula (I) 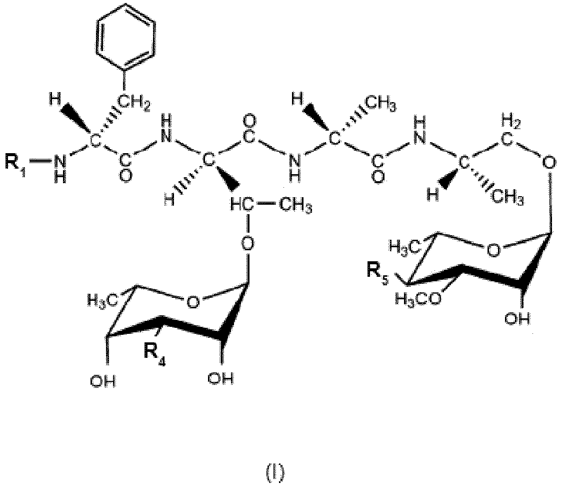
wherein R₁ is 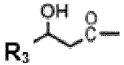
wherein **R₃** is an alkyl or alkenyl chain having from 1 to 34 carbon atoms;
wherein **R₄** is -OH or -OCH_{3;} and
wherein **R₅** is -OH or -OCH_{3;}
for the *in vitro* diagnosis or detection of infections caused not only by *Mycobacterium avium* but caused by any non-tuberculous mycobacteria selected from the group consisting of: *Mycobacterium abcessus, Mycobacterium chimaera, Mycobacterium intracellulare, Mycobacterium malmoense and Mycobacterium xenopi.*

2. Use of *Mycobacterium avium* non-serovar specific GPL antigen of Formula (I) 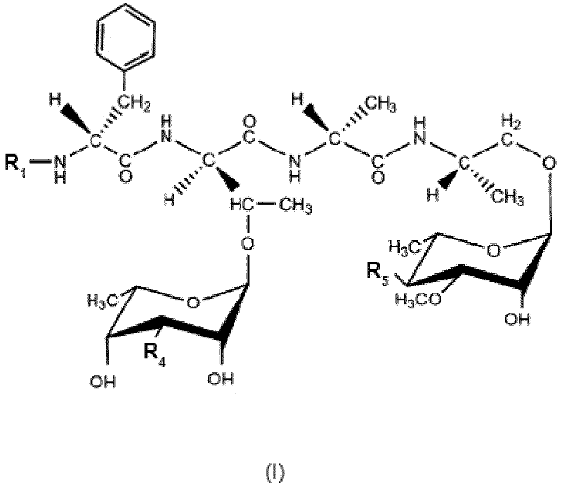
wherein R₁ is 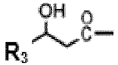
wherein **R₃** is an alkyl or alkenyl chain having from 1 to 34 carbon atoms;
wherein **R₄** is -OH or -OCH_{3;} and
wherein **R₅** is -OH or -OCH_{3;}
for *in vitro* differentiating between infections caused not only by *Mycobacterium avium* but caused by any non-tuberculous mycobacteria selected from the group consisting of: *Mycobacterium abcessus, Mycobacterium chimaera, Mycobacterium intracellulare, Mycobacterium malmoense and Mycobacterium xenopi,* from latent tuberculosis infection.

3. Use of *Mycobacterium avium* non-serovar specific GPL antigen of Formula (I) 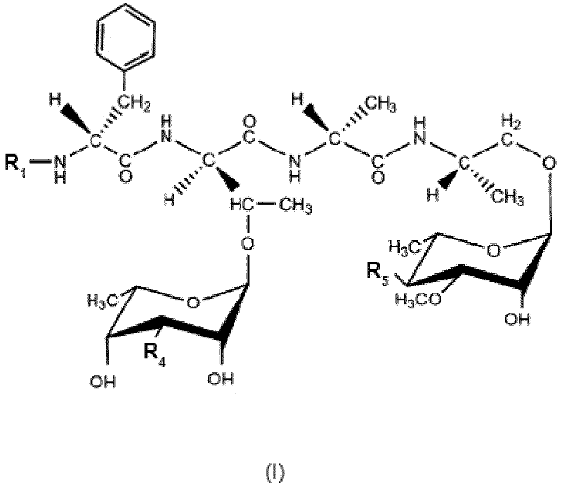
wherein R₁ is 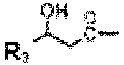
wherein **R₃** is an alkyl or alkenyl chain having from 1 to 34 carbon atoms;
wherein **R₄** is -OH or -OCH_{3;} and
wherein **R₅** is -OH or -OCH_{3;}
for deciding whether to administer a medical treatment to a subject suspected of suffering from an infection caused not only by *Mycobacterium avium* but caused by any non-tuberculous mycobacteria selected from the group consisting of: *Mycobacterium abcessus, Mycobacterium chimaera, Mycobacterium intracellulare, Mycobacterium malmoense and Mycobacterium xenopi.*

4. The use, according to any of the claims 1 to 3, wherein the non-tuberculous mycobacteria infection is identified in subjects suffering from: lymphadenitis, skin and soft tissue infection, chronic pulmonary diseases, and disseminated disease in immune compromised patients.

5. The use, according to any of the claims 1 to 4, wherein, in Formula I, **R₃** is an alkenyl chain having 34 carbon atoms, **R₄** is -OCH₃ and **R₅** is -OCH₃.

6. *In vitro* method for detecting or diagnosing infections caused not only by *Mycobacterium avium* but caused by any non-tuberculous mycobacteria selected from the group consisting of: *Mycobacterium abcessus, Mycobacterium chimaera, Mycobacterium intracellulare, Mycobacterium malmoense and Mycobacterium xenopi,* said method comprising:
a) incubating a biological sample, under the condition that it contains leucocytes, obtained from the subject with *Mycobacterium avium* non-serovar specific GPL antigen of Formula (I) 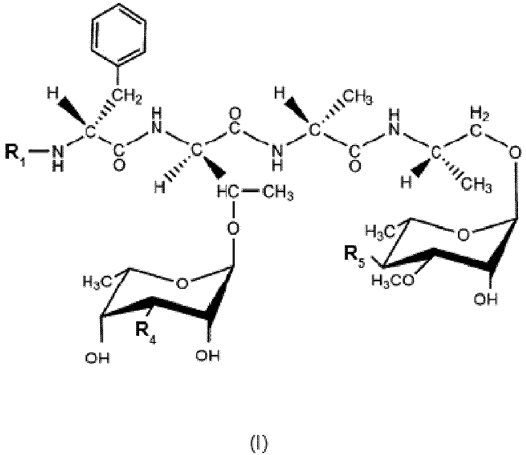
wherein R₁ is 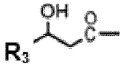
wherein **R₃** is an alkyl or alkenyl chain having from 1 to 34 carbon atoms;
wherein **R₄** is -OH or -OCH_{3;} and
wherein **R₅** is -OH or -OCH_{3;}
b) quantifying the secreted level of at least one cytokine,
c) wherein if the cytokine level quantified in step (b) is higher than the reference control level, it is indicative that the subject is suffering from an infection caused not only by *Mycobacterium avium* but caused by any non-tuberculous mycobacteria selected from the group consisting of: *Mycobacterium abcessus, Mycobacterium chimaera, Mycobacterium intracellulare, Mycobacterium malmoense and Mycobacterium xenopi.*

7. *In vitro* method for differentiating between infections caused not only by *Mycobacterium avium* but caused by any non-tuberculous mycobacteria selected from the group consisting of: *Mycobacterium abcessus, Mycobacterium chimaera, Mycobacterium intracellulare, Mycobacterium malmoense and Mycobacterium xenopi,* from latent tuberculosis infection, said method comprising:
a) Incubating a biological sample, under the condition that it contains leucocytes, obtained from the subject with *Mycobacterium avium* non-serovar specific GPL antigen of Formula (I) 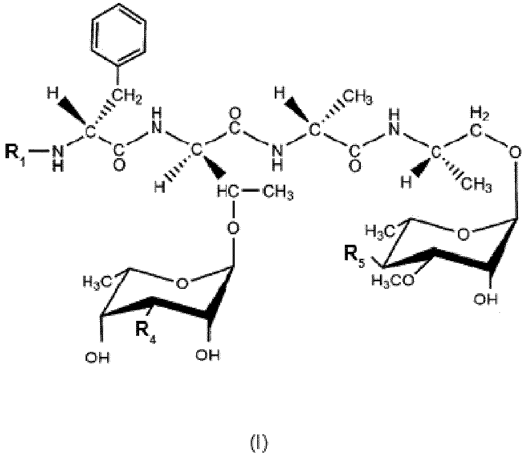
wherein R₁ is 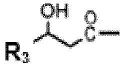
wherein **R₃** is an alkyl or alkenyl chain having from 1 to 34 carbon atoms;
wherein **R₄** is -OH or -OCH_{3;} and
wherein **R₅** is -OH or -OCH_{3;}
b) quantifying the secreted level of at least one cytokine,
c) wherein if the cytokine level quantified in step (b) is higher than the reference control level, it is indicative that the subject is suffering from an infection caused not only by *Mycobacterium avium* but caused by any non-tuberculous mycobacteria selected from the group consisting of : *Mycobacterium abcessus, Mycobacterium chimaera, Mycobacterium intracellulare, Mycobacterium malmoense and Mycobacterium xenopi,* and a latent tuberculosis infection can be discarded when conventional IGRAs test is negative.

8. *In vitro* method for deciding whether to administer a medical treatment to a subject suspected of suffering from an infection caused not only by *Mycobacterium avium* but caused by any non-tuberculous mycobacteria selected from the group consisting of: *Mycobacterium abcessus, Mycobacterium chimaera, Mycobacterium intracellulare, Mycobacterium malmoense and Mycobacterium xenopi,* said method comprising:
a) Incubating a biological sample, under the condition that it contains leucocytes, obtained from the subject with *Mycobacterium avium* non-serovar specific GPL antigen of Formula (I) 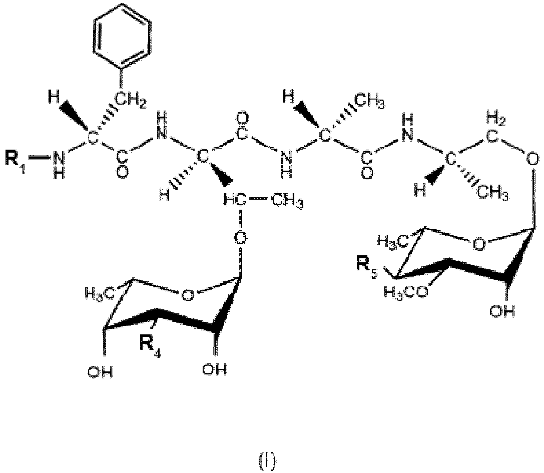
wherein **R₁** is 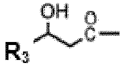
wherein **R₃** is an alkyl or alkenyl chain having from 1 to 34 carbon atoms;
wherein **R₄** is -OH or -OCH_{3;} and
wherein **R₅** is -OH or -OCH_{3;}
b) quantifying the secreted level of at least one cytokine, and
c) wherein if the cytokine level quantified in step (b) is higher than the reference control level, it is indicative that the subject is suffering from an infection caused not only by *Mycobacterium avium* but caused by any non-tuberculous mycobacteria selected from the group consisting of: *Mycobacterium abcessus, Mycobacterium chimaera, Mycobacterium intracellulare, Mycobacterium malmoense and Mycobacterium xenopi,* and a medical treatment directed to NTM could be applied.

9. The method, according to any of the claims 6 to 8, wherein the cytokine or combination of cytokines are selected from the group comprising: IFN-gamma, TNF-alpha, IP-10, IL-10, IL-1 RA, GM-CSF, IL-13, IL-17, IL-5, IL-2, IL-22 and IL-32.

10. The method, according to any of claims 6 to 19, wherein the non-tuberculous mycobacteria infection is identified in subjects suffering from: lymphadenitis, skin and soft tissue infection, chronic pulmonary diseases, disseminated disease in immune compromised patients.

11. The method, according to any of claims 9 to 10, wherein the biological sample is selected from: whole blood, saliva, bronchoalveolar fluids, cerebrospinal fluids, purified peripheral blood mononuclear cells (PBMC), or biopsies.

12. The method, according to any of claims 6 to 11, wherein, in Formula I, **R₃** is an alkenyl chain having 34 carbon atoms, **R₄** is -OCH₃ and **R₅** is -OCH₃.

## Patentansprüche

1. Verwendung vom Antigen gegen vom nicht-serovar spezifischen GPL (nicht-serovar Glykopeptidolipid, nsGPL) von *Mycobacterium avium* der Formel (I) 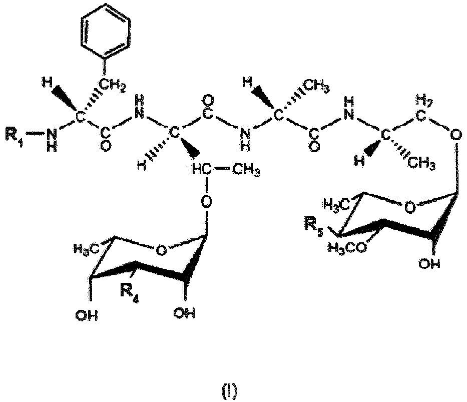
in welcher R₁ 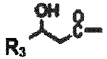 ist;
wobei **R₃** eine Alkyl- oder Alkenylkette aufweisend von 1 bis 34 Kohlenstoffatome ist;
in welcher **R₄** -OH oder -OCH₃ ist; und
in welcher **R₅** -OH oder -OCH₃ ist;
zur In-vitro-Diagnose oder -Detektion von Infektionen hervorgerufen nicht nur von *Mycobacterium avium,* sondern hervorgerufen von jeder nicht tuberkulösen Mykobakterie ausgewählt aus der Gruppe bestehend aus: *Mycobacterium abcessus, Mycobacterium chimaera, Mycobacterium intracellulare, Mycobacterium malmoense* und *Mycobacterium xenopi.*

2. Verwendung vom Antigen vom nicht-serovar spezifischen GPL von *Mycobacterium avium* der Formel (I) 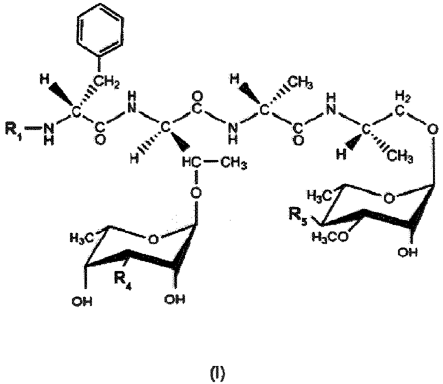
in welcher R₁ 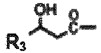 ist,
wobei **R₃** eine Alkyl- oder Alkenylkette aufweisend von 1 bis 34 Kohlenstoffatome ist;
in welcher **R₄** -OH oder -OCH₃ ist; und
in welcher **R₅** -OH oder -OCH₃ ist;
zur In-vitro-Differenzierung zwischen Infektionen hervorgerufen nicht nur von *Mycobacterium avium,* sondern hervorgerufen von jeder nicht tuberkulösen Mykobakterie ausgewählt aus der Gruppe bestehend aus: *Mycobacterium abcessus, Mycobacterium chimaera, Mycobacterium intracellulare, Mycobacterium malmoense* und *Mycobacterium xenopi,* und latenter Tuberkuloseinfektion.

3. Verwendung vom Antigen vom nicht-serovar spezifischen GPL von *Mycobacterium avium* der Formel (I) 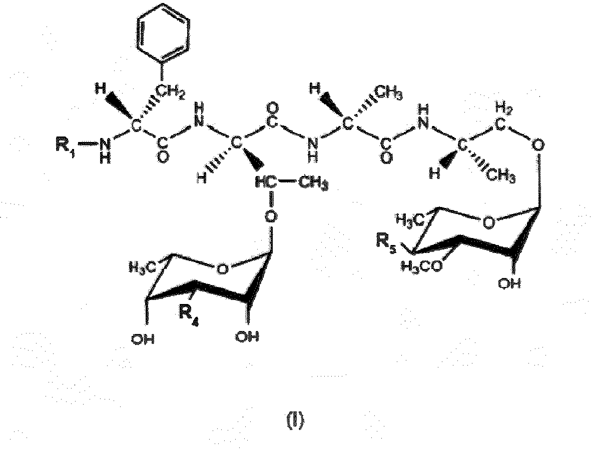
in welcher R₁ 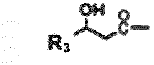 ist,
wobei **R₃** eine Alkyl- oder Alkenylkette aufweisend von 1 bis 34 Kohlenstoffatome ist;
in welcher **R₄** -OH oder -OCH₃ ist; und
in welcher **R₅** -OH oder -OCH₃ ist;
zum Entscheiden, ob eine medizinische Behandlung einem Individuum verabreicht werden soll, von welchem es verdächtigt wird, unter einer Infektion zu leiden, hervorgerufen nicht nur von *Mycobacterium avium,* sondern hervorgerufen von jeder nicht tuberkulösen Mykobakterie ausgewählt aus der Gruppe bestehend aus: *Mycobacterium abcessus, Mycobacterium chimaera, Mycobacterium intracellulare, Mycobacterium malmoense* und *Mycobacterium xenopi.*

4. Verwendung nach einem der Ansprüche 1 bis 3, wobei die nicht tuberkulöse Mykobakterieninfektion in Individuen identifiziert wird, welcher unter den Folgenden leiden: Lymphadenitis, Haut- und Weichteilinfektion, chronischen Lungenerkrankungen und disseminierter Krankheit in immungeschwächten Patienten.

5. Verwendung nach einem der Ansprüche 1 bis 4, wobei, in Formel I, **R₃** eine Alkenylkette aufweisend 34 Kohlenstoffatome ist, **R₄** -OCH₃ ist und **R₅** -OCH₃ ist.

6. In-vitro-Verfahren zum Detektieren oder Diagnostizieren von Infektionen hervorgerufen nicht nur von *Mycobacterium avium,* sondern hervorgerufen von jeder nicht tuberkulösen Mykobakterie ausgewählt aus der Gruppe bestehend aus: *Mycobacterium abcessus, Mycobacterium chimaera, Mycobacterium intracellulare, Mycobacterium malmoense* und *Mycobacterium xenopi,* wobei das genannte Verfahren Folgendes umfasst:
a) das Inkubieren einer biologischen Probe, unter der Bedingung, dass sie Leukozyten enthält, erhalten aus dem Individuum mit Antigen vom nicht-serovar spezifischen GPL von *Mycobacterium avium* der Formel (I) 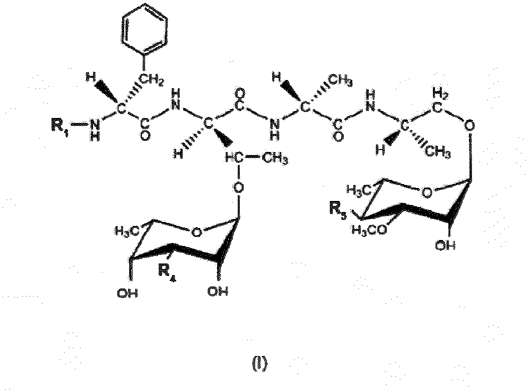
in welcher R₁ 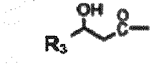 ist,
wobei **R₃** eine Alkyl- oder Alkenylkette aufweisend von 1 bis 34 Kohlenstoffatome ist;
in welcher **R₄** -OH oder -OCH₃ ist; und
in welcher **R₅** -OH oder -OCH₃ ist;
b) das Quantifizieren des sekretierten Niveaus von mindestens einem Zytokin,
c) wobei, wenn das in Schritt (b) quantifizierte Zytokinniveau höher als das Referenzsteuerniveau ist, es darauf schließen lässt, dass das Individuum unter einer Infektion leidet, hervorgerufen nicht nur von *Mycobacterium avium,* sondern hervorgerufen von jeder nicht tuberkulösen Mykobakterie ausgewählt aus der Gruppe bestehend aus: *Mycobacterium abcessus, Mycobacterium chimaera, Mycobacterium intracellulare, Mycobacterium malmoense* und *Mycobacterium xenopi.*

7. In-vitro-Verfahren zur Differenzierung zwischen Infektionen hervorgerufen nicht nur von *Mycobacterium avium,* sondern hervorgerufen von jeder nicht tuberkulösen Mykobakterie ausgewählt aus der Gruppe bestehend aus: *Mycobacterium abcessus, Mycobacterium chimaera, Mycobacterium intracellulare, Mycobacterium malmoense* und *Mycobacterium xenopi,* und latenter Tuberkuloseinfektion, wobei das genannte Verfahren Folgendes umfasst:
a) das Inkubieren einer biologischen Probe, unter der Bedingung, dass sie Leukozyten enthält, erhalten aus dem Individuum mit Antigen vom nicht-serovar spezifischen GPL von *Mycobacterium avium* der Formel (I) 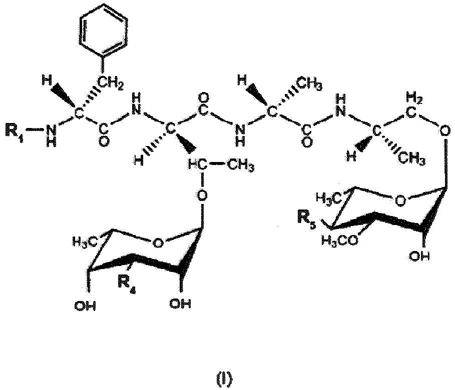
in welcher R₁ 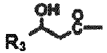 ist,
wobei **R₃** eine Alkyl- oder Alkenylkette aufweisend von 1 bis 34 Kohlenstoffatome ist;
in welcher **R₄** -OH oder -OCH₃ ist; und
in welcher **R₅** -OH oder -OCH₃ ist;
b) das Quantifizieren des sekretierten Niveaus von mindestens einem Zytokin,
c) wobei, wenn das in Schritt (b) quantifizierte Zytokinniveau höher als das Referenzsteuerniveau ist, es darauf schließen lässt, dass das Individuum unter einer Infektion leidet, hervorgerufen nicht nur von *Mycobacterium avium,* sondern hervorgerufen von jeder nicht tuberkulösen Mykobakterie ausgewählt aus der Gruppe bestehend aus: *Mycobacterium abcessus, Mycobacterium chimaera, Mycobacterium intracellulare, Mycobacterium malmoense* und *Mycobacterium xenopi,* und eine latente Tuberkuloseinfektion ausgeschlossen werden kann, wenn der herkömmliche IGRA-Test negativ ist.

8. In-vitro-Verfahren zum Entscheiden ob eine medizinische Behandlung einem Individuum verabreicht werden soll, von welchem es verdächtigt wird, unter einer Infektion zu leiden, hervorgerufen nicht nur von *Mycobacterium avium,* sondern hervorgerufen von jeder nicht tuberkulösen Mykobakterie ausgewählt aus der Gruppe bestehend aus: *Mycobacterium abcessus, Mycobacterium chimaera, Mycobacterium intracellulare, Mycobacterium malmoense* und *Mycobacterium xenopi,* wobei das genannte Verfahren Folgendes umfasst:
a) das Inkubieren einer biologischen Probe, unter der Bedingung, dass sie Leukozyten enthält, erhalten aus dem Individuum mit Antigen vom nicht-serovar spezifischen GPL von *Mycobacterium avium* der Formel (I) 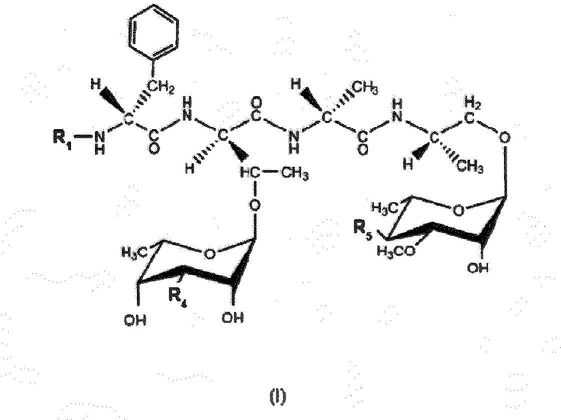
in welcher R₁ 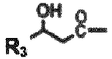 ist,
wobei **R₃** eine Alkyl- oder Alkenylkette aufweisend von 1 bis 34 Kohlenstoffatome ist;
in welcher **R₄** -OH oder -OCH₃ ist; und
in welcher **R₅** -OH oder -OCH₃ ist;
b) das Quantifizieren des sekretierten Niveaus von mindestens einem Zytokin, und
c) wobei, wenn das in Schritt (b) quantifizierte Zytokinniveau höher als das Referenzsteuerniveau ist, es darauf schließen lässt, dass das Individuum unter einer Infektion leidet, hervorgerufen nicht nur von *Mycobacterium avium,* sondern hervorgerufen von jeder nicht tuberkulösen Mykobakterie ausgewählt aus der Gruppe bestehend aus: *Mycobacterium abcessus, Mycobacterium chimaera, Mycobacterium intracellulare, Mycobacterium malmoense* und *Mycobacterium xenopi,* und eine medizinische Behandlung gegen NTM gerichtet angewendet werden könnte.

9. Verfahren nach einem der Ansprüche 6 bis 8, wobei das Zytokin oder die Kombination von Zytokinen aus der Gruppe umfassend: IFN-gamma, TNF-alpha, IP-10, IL-10, IL-1 RA, GM-CSF, IL-13, IL-17, IL-5, IL-2, IL-22 und IL-32, ausgewählt werden.

10. Verfahren nach einem der Ansprüche 6 bis 19, wobei die nicht tuberkulöse Mykobakterieninfektion in Individuen identifiziert wird, welche unter den Folgenden leiden: Lymphadenitis, Haut- und Weichteilinfektion, chronischen Lungenerkrankungen, disseminierter Krankheit in immungeschwächten Patienten.

11. Verfahren nach einem der Ansprüche 9 bis 10, wobei die biologische Probe aus den Folgenden ausgewählt wird: Vollblut, Speichel, bronchoalveolaren Flüssigkeiten, Gehirn-Rückenmark-Flüssigkeiten, gereinigten mononukleären Zellen des peripheren Bluts (PBMC) oder Biopsien.

12. Verfahren nach einem der Ansprüche 6 bis 11, wobei, in Formel I, R₃ eine Alkenylkette aufweisend 34 Kohlenstoffatome ist, R₄ -OCH₃ ist und R₅ -OCH₃ ist.

## Revendications

1. Utilisation d'antigène GPL non-spécifique de sérovar de *Mycobacterium avium* (glycopeptidolipide non-sérovar, nsGPL) de formule (I) 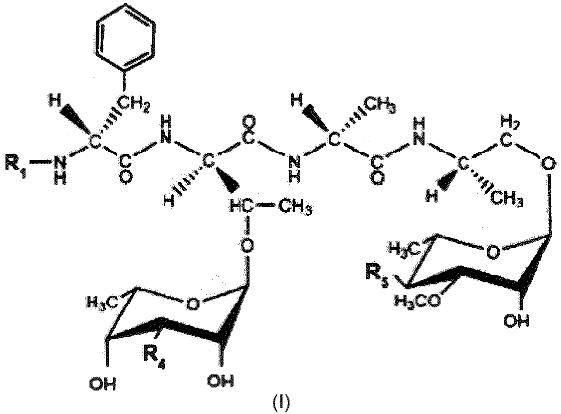
dans laquelle R₁ est 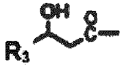
dans laquelle R₃ est une chaîne alkyle ou alcényle ayant de 1 jusqu'à 34 atomes de carbone ;
dans laquelle R₄ est -OH ou -OCH₃ ; et
dans laquelle R₅ est -OH ou -OCH₃ ;
pour le diagnostic ou la détection *in vitro* d'infections causées non seulement par *Mycobacterium avium* mais causées par une quelconque mycobactérie non tuberculeuse sélectionnée du groupe consistant en : *Mycobacterium abcessus, Mycobacterium chimaera, Mycobacterium* intracellulare, *Mycobacterium malmoense et Mycobacterium xenopi.*

2. Utilisation d'antigène GPL non-spécifique de sérovar de *Mycobacterium avium* de formule (I) 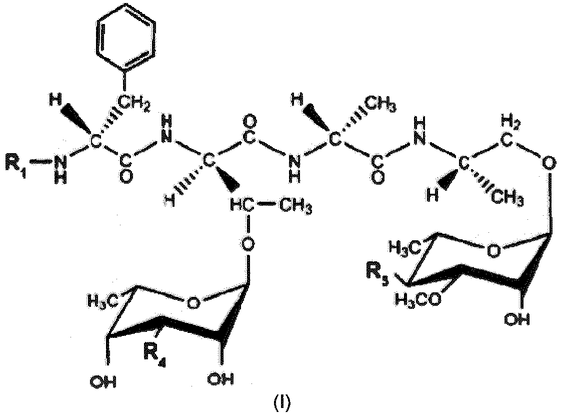
dans laquelle R₁ est 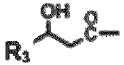
dans laquelle R₃ est une chaîne alkyle ou alcényle ayant de 1 jusqu'à 34 atomes de carbone ;
dans laquelle R₄ est -OH ou -OCH₃ ; et
dans laquelle R₅ est -OH ou -OCH₃ ;
pour la différenciation *in vitro* entre infections causées non seulement par *Mycobacterium avium* mais causées par une quelconque mycobactérie non tuberculeuse sélectionnée du groupe consistant en : *Mycobacterium abcessus, Mycobacterium chimaera, Mycobacterium intracellulare, Mycobacterium malmoense et Mycobacterium xenopi,* à partir d'une infection tuberculeuse latente.

3. Utilisation d'antigène GPL non-spécifique de sérovar de *Mycobacterium avium* de formule (I) 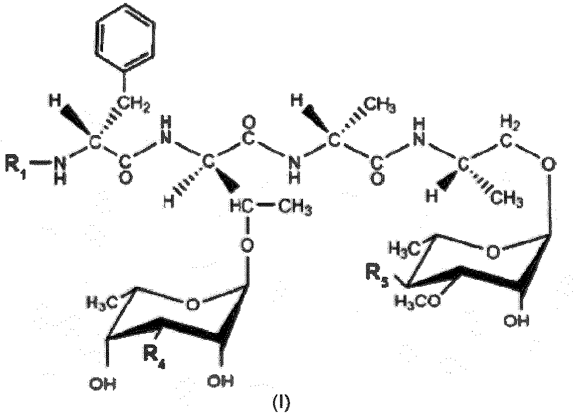
dans laquelle R₁ est 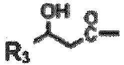
dans laquelle R₃ est une chaîne alkyle ou alcényle ayant de 1 jusqu'à 34 atomes de carbone ;
dans laquelle R₄ est -OH ou -OCH₃ ; et
dans laquelle R₅ est -OH ou -OCH₃ ;
pour décider si administrer un traitement médical à un sujet soupçonné de souffrir d'une infection causée non seulement par *Mycobacterium avium* mais causée par une quelconque mycobactérie non tuberculeuse sélectionnée du groupe consistant en : *Mycobacterium abcessus, Mycobacterium chimaera, Mycobacterium intracellulare, Mycobacterium malmoense et Mycobacterium xenopi.*

4. Utilisation selon l'une quelconque des revendications 1 à 3, dans laquelle l'infection à mycobactéries non tuberculeuses est identifiée chez des sujets souffrant de : lymphadénite, infection de peau et de tissus mous, maladies pulmonaires chroniques et maladies disséminées chez des patients immunodéficients.

5. Utilisation selon l'une quelconque des revendications 1 à 4, dans laquelle, dans la formule I, R₃ est une chaîne alcényle ayant 34 atomes de carbone, R₄ est -OCH₃ et R₅ est -OCH₃.

6. Procédé *in vitro* pour détecter ou diagnostiquer des infections causées non seulement par *Mycobacterium avium* mais causées par une quelconque mycobactérie non tuberculeuse sélectionnée du groupe consistant en : *Mycobacterium abcessus, Mycobacterium chimaera, Mycobacterium intracellulare, Mycobacterium malmoense et Mycobacterium xenopi,* ledit procédé comprenant :
a) incuber un échantillon biologique, sous la condition qu'il contienne des leucocytes, obtenu du sujet avec l'antigène GPL non-spécifique de sérovar de *Mycobacterium avium* de formule (I) 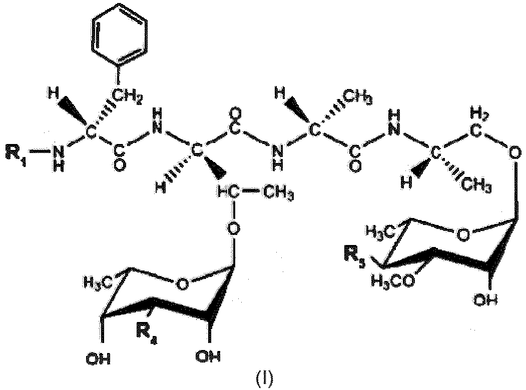
dans laquelle R₁ est 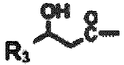
dans laquelle R₃ est une chaîne alkyle ou alcényle ayant de 1 jusqu'à 34 atomes de carbone ;
dans laquelle R₄ est -OH ou -OCH₃ ; et
dans laquelle R₅ est -OH ou -OCH₃ ;
b) quantifier le niveau sécrété d'au moins une cytokine,
c) dans lequel si le niveau de cytokine quantifié dans l'étape (b) est plus élevé que le niveau de contrôle de référence, cela est révélateur que le sujet souffre d'une infection causée non seulement par *Mycobacterium avium* mais causée par une quelconque mycobactérie non tuberculeuse sélectionnée du groupe consistant en : *Mycobacterium abcessus, Mycobacterium chimaera, Mycobacterium* intracellulare, *Mycobacterium malmoense et Mycobacterium xenopi.*

7. Procédé *in vitro* pour différencier entre les infections causées non seulement par *Mycobacterium avium* mais causée par une quelconque mycobactérie non tuberculeuse sélectionnée du groupe consistant en : *Mycobacterium abcessus, Mycobacterium chimaera, Mycobacterium intracellulare, Mycobacterium malmoense et Mycobacterium xenopi,* d'une infection tuberculeuse latente, ledit procédé comprenant :
a) incuber un échantillon biologique, sous la condition qu'il contienne des leucocytes, obtenu du sujet avec l'antigène GPL non-spécifique de sérovar de *Mycobacterium avium* de formule (I) 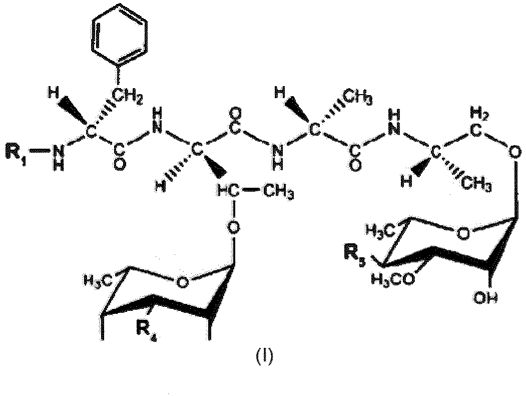
dans laquelle R₁ est 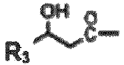
dans laquelle R₃ est une chaîne alkyle ou alcényle ayant de 1 jusqu'à 34 atomes de carbone ;
dans laquelle R₄ est -OH ou -OCH₃ ; et
dans laquelle R₅ est -OH ou -OCH₃ ;
b) quantifier le niveau sécrété d'au moins une cytokine,
c) dans lequel si le niveau de cytokine quantifié dans l'étape (b) est plus élevé que le niveau de contrôle de référence, cela est révélateur que le sujet souffre d'une infection causée non seulement par *Mycobacterium avium* mais causée par une quelconque mycobactérie non tuberculeuse sélectionnée du groupe consistant en : *Mycobacterium abcessus, Mycobacterium chimaera, Mycobacterium intracellulare, Mycobacterium malmoense et Mycobacterium xenopi,* et une infection tuberculeuse latente peut être rejetée lorsque le test IGRA conventionnel est négatif.

8. Procédé *in vitro* pour décider si administrer un traitement médical à un sujet soupçonné de souffrir d'une infection causée non seulement par *Mycobacterium avium* mais causée par une quelconque mycobactérie non tuberculeuse sélectionnée du groupe consistant en : *Mycobacterium abcessus, Mycobacterium chimaera, Mycobacterium intracellulare, Mycobacterium malmoense et Mycobacterium xenopi,* ledit procédé comprenant :
a) incuber un échantillon biologique, sous la condition qu'il contienne des leucocytes, obtenu du sujet avec l'antigène GPL non-spécifique de sérovar de *Mycobacterium avium* de formule (I) 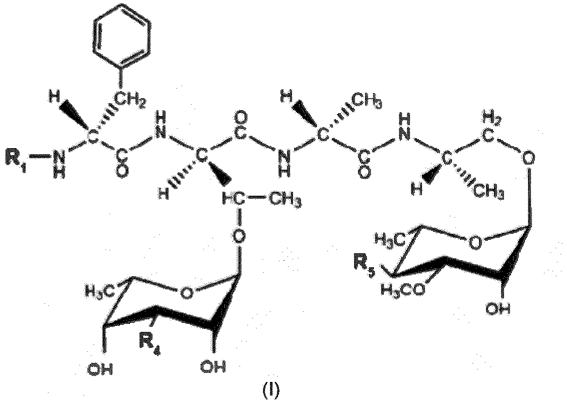
dans laquelle R₁ est 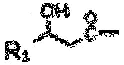
dans laquelle R₃ est une chaîne alkyle ou alcényle ayant de 1 jusqu'à 34 atomes de carbone ;
dans laquelle R₄ est -OH ou -OCH₃ ; et
dans laquelle R₅ est -OH ou -OCH₃ ;
b) quantifier le niveau sécrété d'au moins une cytokine, et
c) dans lequel si le niveau de cytokine quantifié dans l'étape (b) est plus élevé que le niveau de contrôle de référence, cela est révélateur que le sujet souffre d'une infection causée non seulement par *Mycobacterium avium* mais causée par une quelconque mycobactérie non tuberculeuse sélectionnée du groupe consistant en : *Mycobacterium abcessus, Mycobacterium chimaera, Mycobacterium* intracellulare, *Mycobacterium malmoense et Mycobacterium xenopi,* et un traitement médical dirigé vers les MNT peut être appliqué.

9. Procédé selon l'une quelconque des revendications 6 à 8, dans lequel la cytokine ou la combinaison de cytokines sont sélectionnées du groupe comprenant: IFN-gamma, TNF-alpha, IP-10, IL-10, IL-1RA, GM-CSF, IL-13, IL-17, IL-5, IL-2, IL-22 et IL-32.

10. Procédé selon l'une quelconque des revendications 6 à 19, dans lequel l'infection à mycobactéries non tuberculeuses est identifiée chez des sujets souffrant de : lymphadénite, infection de peau et de tissus mous, maladies pulmonaires chroniques, maladies disséminées chez des patients immunodéficients.

11. Procédé selon l'une quelconque des revendications 9 à 10, dans lequel l'échantillon biologique est sélectionné de : sang total, salive, fluides broncho-alvéolaires, fluides cérébro-spinaux, cellules mononucléaires du sang périphérique (PBMC) purifiées ou biopsies.

12. Procédé selon l'une quelconque des revendications 6 à 11, dans lequel, dans la formule I, R₃ est une chaîne alcényle ayant 34 atomes de carbone, R₄ est -OCH₃ et R₅ est -OCH₃.
